# EUROPEAN PATENT SPECIFICATION

(11) **EP 4 376 772 B1**
(45) Date of publication and mention of the grant of the patent: **10.06.2026**
(21) Application number: 22761592.9
(22) Date of filing: 25.07.2022
(51) Int. Cl.: A61F 2/844, A61F 2/24, A61F 2/966

(54) **VALVE PROSTHESIS AND TRANSCATHETER DELIVERY SYSTEM**
KLAPPENPROTHESE UND TRANSKATHETEREINFÜHRUNGSSYSTEM
PROTHÈSE DE VALVULE ET SYSTÈME DE POSE PAR TRANSCATHÉTER

(30) Priority: 27.07.2021 US 202163225969 P; 18.02.2022 US 202263311650 P
(43) Date of publication of application: 05.06.2024
(73) Proprietor: Open Stent Solution (SAS), 80480 Dury (FR)
(72) Inventor: LOMBARDI, Fabien, 80480 Dury (FR); PARSAIX, Stephane, 80480 Dury (FR); BORDJI, Mehdi, 80480 Dury (FR); HARIM, Ayoub, 80480 Dury (FR); LEROY, Matthieu, 80480 Dury (FR)
(74) Representative: Vienne, Aymeric Charles Emile
(86) International application number: PCT/IB2022/000431
(87) International publication number: WO 2023/007245

(56) References cited:
- WO-A1-2019/003221
- WO-A1-2021/055266
- WO-A2-2008/100382
- US-A1- 2020 297 491

## Description

### CROSS-REFERENCE TO RELATED APPLICATIONS

This PCT application claims priority to U.S. Provisional Application 63/311,650, filed on February 18, 2022 and to U.S. Provisional Application 63/225,969, filed on July 27, 2021.

### FIELD OF THE INVENTION

The present disclosure relates generally to medical implants and to methods and systems for delivery of medical implants into a human body. More particularly, the present disclosure relates to methods and systems for the trans-catheter delivery of cardiac valve implants to a human heart.

### BACKGROUND OF THE INVENTION

Heart Valve Disease (HVD) affects many people globally. HVD can manifest in abnormal valve leaflet tissue in various ways, including excess tissue growth, tissue degradation, tissue rupture, tissue hardening, tissue calcification, abnormal tissue re-positioning in response to cardiac configuration during different stages of the cardiac cycle, for example annular dilation or ventricular reshaping. Such abnormal tissue often leads to degradation of valve function, for example, leakage, backflow as a result of valve insufficiency, resistance to blood forward flow as a result of valve stenosis, and the like.

In such situations the best treatment mode is generally replacement of the failing native valve with an artificial prosthetic valve. A prosthetic valve generally provides a functional replacement of a damaged heart valve. In recent years, a preferred mode of delivery of placement of a prosthetic valve has been via catheterization techniques.

Catheter-based procedures are commonly used to treat patients suffering from failing native valve who are not candidates for open surgical procedure or wherein access with minimally invasive surgery is preferable. In such catheter-based procedure, a stent and/or prosthetic valve may be delivered to a human heart using a percutaneous approach. In such delivery systems a prosthetic device, for example a stent and/or prosthetic valve or the like vessel support structures are mounted on a balloon catheter and/or a self-expandable device that is advanced to the delivery site and/or location. At the delivery site a prosthetic device, for example a valvular device, is deployed by outwardly expanding the balloon from the distal end of the delivery system or retrieve the sheath in order to expand the self-expandable device. Therefore, the balloon is used to cause the prosthetic device to expand centrally, from the center out, so as to allow the deployment of the prosthetic device, for example a prosthetic valvular device, at the delivery site. Such central expansion is further limited for situations where large diameter valves are required for example where the native valve annulus is extremely dilated. In such situations state of the art delivery systems become very large (diameter) therefore limiting the possibility of navigation through the vasculature and further limiting the possibility of a trans-femoral trans-septal approach.

Furthermore, current delivery systems have to be adapted according to the dimensions of the prosthetic device, balloon expandable device or self-expandable device- In the case of atrioventricular (AV) valve replacements that use large valve devices, the traditional radial crimp has reached its limits thus forcing the delivery to use the apical approach

The left atrioventricular (AV valve) valve, also called the Mitral valve, poses unique anatomical obstacles, rendering percutaneous mitral valve replacement significantly more challenging. The mitral valve's annulus has a non-circular D-shape or kidney-like shape, with a non-planar, saddle-like, geometry that often lacks symmetry. Such anatomical variation and non-symmetry makes it difficult to deliver a centrally expanding replacement valve utilizing a centrally expanding distal tip as is the current state of the art approach. In addition, anchoring the device by capturing the leaflets with hooks or loops is quite hazardous and prone to failure.

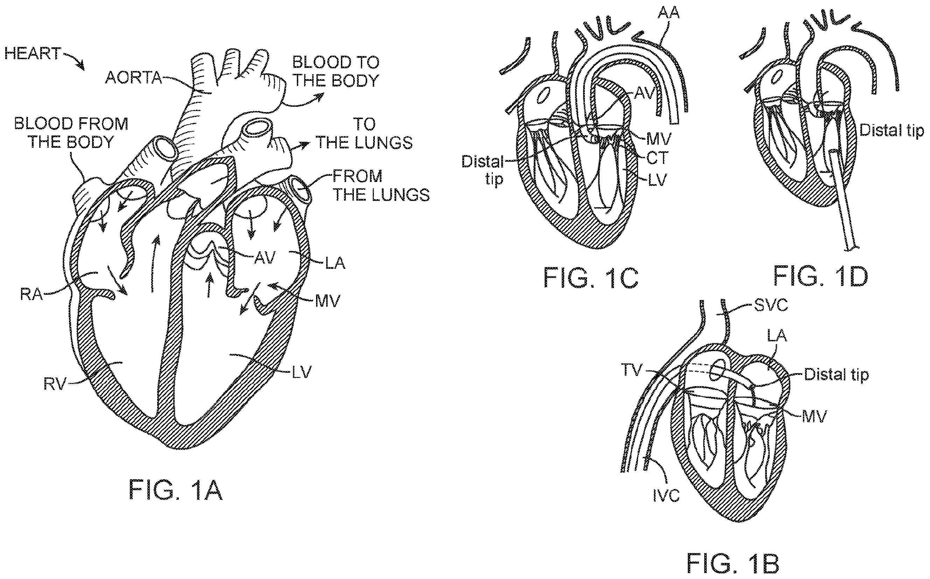

FIG. 1A shows the anatomy of a normal heart with the arrows depicting the flow through the valves. The heart comprises a left atrium (LA) that receives oxygenated blood from the lungs via the pulmonary veins (PV) and pumps this oxygenated blood through the mitral valve (MV) into the left ventricle (LV). When the left ventricle (LV) contracts, allowing blood to flow outwardly through the aortic valve (AV) in the direction of the arrows.

The mitral valve (MV) comprises a pair of leaflets that meet evenly, or "coapt" to close to (MV). The ventricular side of the leaflets are attached to the surrounding heart structures of the left ventricle via an annular region of tissue referred to as the annulus (AN) found on the left atrium. The annulus is a fibrous ring of dense connective tissue which is distinct from both the leaflet tissue as well as the adjoining muscular tissue of the heart wall.

Access to the mitral valve or other atrioventricular valve can be accomplished through the patient's vasculature in a percutaneous manner. By percutaneous it is meant that a location of the vasculature remote from the heart is accessed through the skin, typically using a surgical cut down procedure or a minimally invasive procedure. The ability to percutaneously access the remote vasculature is well-known and described in the patent and medical literature. Depending on the point of vascular access, the approach to the mitral valve may be antegrade and may rely on entry into the left atrium by crossing the inter-atrial septum, as shown in FIG. 1B, this approach is also called the trans-septal approach. Alternatively, approach to the mitral valve can be retrograde where the left ventricle is entered through the aortic valve, for example as shown in FIG. 1C. An additional retrograde approach may be provided by a trans-apical puncture where access to the mitral valve is gained via a puncture at or near the apex of the heart, as shown in FIG. 1D.

Once access to the heart is achieved, the interventional tools and supporting catheter(s) may be advanced to the heart and positioned adjacent the target cardiac to allow for the deployment of the necessary prosthetic devices. In all such approaches the functional end of the delivery system of the state of the art is found at the distal end and/or distal tip of the functional tools for example, catheter(s), tool(s) used to deliver the prosthetic device(s).

Generally, state of the art delivery systems comprises a guidewire to introduce a guide catheter and the prosthetic device. After placing a guidewire, the guide catheter may be introduced over the guidewire to the desired position and treatment site. WO 2008/100382 discloses a monolithic in-situ forming valve system. WO 2019/003221 A1 relates to an intraluminal support structure having a delivery configuration that is a crimped open configuration to increase flexibility while maneuvering in the anatomy and having a small scarring signature.

### St MMARY OF THE INVENTION

Trans-catheter therapies for structural heart diseases raised the need for the delivery of large devices through native vessels, cardiac walls and the cardiac septum. However, radial crimping of these devices, as provided by the state-of-the-art devices, creates a rigid and relatively large stem inside the device's delivery sheath. These limitations, both in rigidity and size, result in the limited movement and maneuverability during the procedure. In particular it is difficult to maneuver the device in acute angles during the procedure.

Furthermore, the large size (diameter) of the state-of-the-art device further leave large orifices in the cardiac tissue, particularly the septum, at the end of the procedure.

In state of art, all the devices are crimped loaded and deployed in a radial manner, from the distal tip and/or end of the delivery device. This means that currently there isn't any available option to deliver prosthetic devices, such as stents and/or valves, without significant septum damage that are further limited in maneuverability.

There is an unmet need for, and it would be highly useful to have, a stent that is more flexible in its crimped state so as to facilitate delivery in any anatomy and to allow for easier maneuverability through the tortuous anatomy. Accordingly, increased stent flexibility greatly increases the capability of delivery and deployment of stents.

While flexibility is important stents must also exhibit high scaffolding in the expanded and/or non-crimped state so as to increase the stability of the stent within the delivery site.

This dual need for flexibility in the crimped state and high scaffolding in the expanded (non-crimped) state presents an unsolved problem in the art as the two characteristics are inversely proportional. Specifically, as stent flexibility is increased, scaffolding is decreased and similarly, as scaffolding is increased, flexibility is decreased. Accordingly, there remains a need for a stent and a corresponding delivery system having a high degree of flexibility in the crimped low-profile state and high scaffolding in the expanded final state.

The prior art discloses cylindrical stents that are convenient for catheterization delivery in that they can assume a small diameter and can be readily expanded with a balloon or alternatively may be configured to be self-expanding. However, due to the cylindrical configuration of stents there is an inherent structural limitation in the level of available flexibility, especially of larger stents configured to form valve prosthesis.

Trans-catheter delivery solution for the treatment of valvular disease wherein, valve replacement is provided by way of catheter facilitated delivery, has further raised the need to deliver a relatively large stents integrated with its biological component, forming a prosthetic valve. Prosthetic valves are introduced to the cardiac anatomy through native vessels, cardiac walls and septum. Accordingly, large stents forming a valve prosthesis, for the purpose of valve delivery, require a high degree of flexibility to facilitate the catheter delivery process.

To this end, state of the art stents are difficult to maneuver within the delivery catheter because of their length and relatively large diameter makes them difficult to maneuver in the tortuous anatomy. Furthermore, the large stent diameter, when in crimped or low profile configures, makes it difficult for the required maneuverability. Specifically establishing acute angels during the delivery is currently not possible with state-of-the-art prosthetic valves, due to the diameter of the cylindrical stent. Furthermore, during trans-catheter delivery the prosthetic valve stent is introduced through the cardiac septum. Current large diameter stents disadvantageously leave a large orifice in the cardiac septum.

Accordingly, it would be advantageous to have a stent capable of increased flexibility while maintaining minimal dimensions in the crimped/small configuration.

The present invention is as set forth in the appended claims. Present disclosures provide a delivery system for a prosthetic device, for example including but not limited to a stent and/or a valve and/or a vessel support structure, having a planar configuration utilized during the delivery phase wherein the prosthetic device assumes a low profile crimped open configuration. The planar stent is configured to assume a final closed folded configuration during deployment at the tissue targeted site.

In embodiments the planar stent may be fit with a valve prosthesis to form a valve prosthesis that may be delivered by trans-catheterization.

In one embodiment, the invention is directed to stents comprising a plurality of interconnected cells where at least one of the interconnected cells is a lockable cell. The lockable cell includes a first locking member and a second locking member disposed opposite the first member. The first and second locking members are movable between a first position in which they do not lock with one another to a position in which they lock with one another.

Within the context of this application the term "prosthetic device" substantially refers to any prosthetic that may be delivered to a delivery site for example including but not limited to a vessel support structure, a prosthetic valve, a stent. The prosthetic device is generally delivered via a guidewire-based delivery system in a crimped small profile configuration to an implantation site with a delivery tool and/or system such as a catheter and/or guidewire-based system. At the delivery site the prosthetic device may be expanded to its "in-use" dimension.

Within the context of this application the term "open crimped configuration" substantially refers to a crimped small profile configuration of the prosthetic device in its delivery state that is to be delivered to an implantation site with a delivery tool such as a catheter. The term "open" refers to a non-tubular or non-cylindrical structure, for example such as a stent or valve. Therein the term "open crimped configuration" refers to a non-radially crimped prosthetic device and/or a support structure.

Within the context of this application the term "open non-crimped planar and/or pre-curved configuration" substantially refers to a configuration of the prosthetic device and/or support structure in its flat and/or pre-curved planar and preferably single layer configuration having maximal dimension prior to being crimped.

Within the context of this application, the term "open" refers to a configuration of the prosthetic device and/or support structure refers to a non-tubular or non-cylindrical structure, for example such as a stent or valve. The term "open" may be utilized to interchangeably refer to a single layer planar structure that is substantially flat and/or pre-curved or to a multilayered planar structure that is substantially flat and/or pre-curved.

Within the context of this application, the phrase "closed, non-planar folded configuration" refers to the final configuration of the prosthetic device and/or a support structure, as would be placed within the anatomy, for example in the form of a tubular stent and/or cylindrical valve. The closed configuration therefore refers to the final shape and/or state of the prosthetic device and/or a vessel support structure following its transformation.

In embodiments of the present invention comprises a delivery system for delivering and deploying a prosthetic device within the circulatory system, and more preferably in the heart, and most preferably in the mitral valve.

Unless otherwise defined, all technical and scientific terms used herein have the same meaning as commonly understood by one of ordinary skill in the art to which this invention belongs. The materials, methods, and examples provided herein are illustrative only and not intended to be limiting.

Implementation of the method and system of the present invention involves performing or completing certain selected tasks or steps manually, automatically, or a combination thereof.

While the present description and figures depict a three-leaflet valve, however embodiments of the present invention are not limited to such a valve configuration and may be accordingly be configured to form any valve type with any number of leaflets.

In a first preferred aspect, the present invention provides an intraluminal support structure comprising a scaffold panel which, when flattened, has a first free and a second free end with a longitudinal axis therebetween. The panel will typically, but not necessarily, have a rectangular periphery with a length in the longitudinal direction and a width in a traverse direction normal to the longitudinal direction. In other instances, the periphery of the flattened panel could be shaped as a parallelogram with the ends shaped to meet and be connected when the panel is circularized.

The scaffold panel is typically configured to be helically wrapped over an elongated carrier region of a delivery catheter, and the first and second free ends are typically configured to be coupled to each other to form (circularize) the scaffold panel into a ring or cylinder. In most instances, the first and second free ends will be coupled before the intraluminal support structure is released from delivery catheter, but in other instances the scaffold panel may be released from the elongated carrier region of the delivery catheter after or simultaneously with release of the intraluminal support structure from the delivery catheter.

Usually, the scaffold panel is formed into the desired ring or cylindrical configuration by the application of external forces, typically using coupling structures on the delivery catheter as described in more detail below. In most instances, delivery catheter is used to transition the scaffold panel from a delivery configuration (as constrained or otherwise held on the delivery catheter during delivery) into the final ring or cylindrical configuration. The scaffold panel is usually pre-shaped as a ring but in some instances may be pre-shaped as a flat panel or have some other programmed or memory shape or configuration. When pre-shaped as a ring or a cylinder, the shape memory of the scaffold panel helps to recover the deployed cylindrical shape. Such shape transition is aided by pushing and usually twisting the proximal end of the scaffold to advance a distal portion of the scaffold panel from a restraining sheath on the delivery catheter, allowing the scaffold panel to recover its cylindrical configuration while a distal end of the panel remains fixed to a distal coupler on the delivery catheter.

In some embodiments, the first free end of the scaffold panel may comprise a first coupling structure and the second free end of the scaffold panel may comprise a second coupling structure. The first and second coupling structures are typically configured to mate or join with each other to form the ring before (or in some instances after) the scaffold panel is released from the elongated carrier region of the delivery catheter.

In some embodiments, the first and second coupling structures may be configured to be coupled by axial engagement controlled by the delivery catheter.

In some embodiments, the first and second coupling structures may comprise one or more of slots, hooks, buttons, snaps, ratchets, and the like.

In most instances, the intraluminal support structure of the present invention further comprises a leaflet structure attached or attachable to an inner surface of the scaffold panel. The leaflet structure typically comprises a plurality of leaflets and is typically connectable to plurality of commissure posts on the scaffold panel. The leaflet structure may have any one of a variety of structures, but will usually comprise, consist of, or consist essentially of two, three, or more separate leaflets which may be independently attached to the scaffold. In preferred instances, the leaflets each comprise a skirt portion and a fin portion, as discussed in more detail below.

In specific instances, one or more of the plurality of commissure of the leaflet structure may be split, e.g. where one commissure has a first segment attached to the first coupling structure of the and a second segment attached to the second coupling structure. In this way, the first segment and the second segment may be separated when the scaffold panel is open (prior to ring formation) and may be configured to be brought together when the first and second coupling structures are attached to form the scaffold panel into the ring. In these instances, the remaining commissures are fixed to one or more commissure posts distributed longitudinally and disposed laterally across the inner face of the scaffold panel when in this flattened configuration. When the panel is in the ring configuration with the free ends of the scaffold joined, the commissural posts will be distributed circumferentially around the scaffold panel, typically having two "fixed" commissural posts in addition to the coupling structures which form a third commissural post when the coupling structures are attached for tricuspid valves.

In preferred instances, the leaflet structure comprises a plurality of coapting leaflets centrally positioned in a frame, wherein the leaflets are sufficiently pliable to open and close in response to hemodynamic forces and wherein the frame is sufficiently stiff to at least partially resist deformation resulting from hemodynamic forces after implantation.

In still further preferred instances, the frame of the valve structure comprises a skirt having an outer periphery attached to an atrial or upstream end of the scaffold panel and plurality of fins extending radially outwardly from the commissures of the prosthetic leaflets, where at least one of the fins may be split into segments which attach the first and second segments of the split fin to the first and second coupling structures of the scaffold panel. Often, each of the fin structures will be split and separately attached to the commissural posts of the scaffold panel.

Supporting the leaflets with a frame comprising a skirt and plurality of fins has a number of advantages, particularly including mechanical isolation of the valve leaflets which reduces stress on the leaflet commissures from the heart contractions. Stress failure of prosthetic leaflet commissures is problematic. The frame also reduces the effective leaflet diameter which allows a lower prosthetic valve height. A lower prosthetic mitral valve height will interfere less with function of the left ventricular outflow tract than previously proposed prosthetic valves having higher valve heights.

In still other preferred instances, when used for mitral valve replacement, the scaffold panel of the luminal support structures of the present invention will have a length in the longitudinal direction in a range from 90 mm to 190 mm and width in a lateral direction in a range from 6 mm to 30 mm and the ring will have a diameter in a range from 30 mm to 60 mm when formed. As the leaflets are spaced radially inwardly from the intraluminal support structure by the skirt, the leaflets will typically have a smaller diameter in a range from 27 mm to 29 mm when the ring is formed.

In a second preferred aspect, the present invention provides a system for implanting a support structure in a valve annulus. The system may comprise any one of the intraluminal support structures described previously in combination with a delivery catheter having a proximal end, a distal end, and an elongated carrier region near the distal end. The system further includes a distal coupler configured to releasably attach the free first end of the scaffold panel and a proximal coupler configured to releasably attach the second end of the scaffold panel, where the distal and proximal couplers are configured to releasably hold the scaffold panel in a helically wrapped configuration within the elongated carrier region prior to release the form the ring configuration.

In preferred instances, the distal coupler may be configured to both translate along and rotate about a longitudinal axis of the catheter. Typically, the delivery catheter comprises an outer shaft and an inner shaft coaxially disposed within a central passage of the outer shaft, where the inner shaft is mounted to both translate and rotate within the central passage. The distal coupler may be carried on a distal region of the inner shaft and the proximal coupler may be carried on a distal region of the outer shaft.

In a third preferred aspect, the present invention provides a method for implanting a support structure in a valve annulus. The method comprises providing any one of the intraluminal support structures and helically wrapping the scaffold panel over an elongated carrier region of a delivery catheter. A distal end of the delivery catheter is advanced to the valve annulus, and the scaffold panel is deployed from the elongated carrier region of the delivery catheter, allowing the scaffold panel radially to transition to form a ring or cylinder within the valve annulus. Usually, but not necessarily, free ends of the panel are attached before the scaffold panel is released from the delivery catheter. In preferred instances, the valve annulus is a cardiac valve selected from the group consisting of a mitral valve, an aortic valve, a pulmonary valve, and a tricuspid valve.

Usually, the scaffold panel is pre-shaped as a ring and deploying the scaffold panel comprises allowing the scaffold panel to transition into the ring or cylinder (although usually the delivery catheter will control both in the panel during the transition). Typically, the delivery catheter twists and axially compresses the free ends of the scaffold panel to effect or assist the transition of scaffold panel into the ring.

In preferred instances, coupling structures on the first and second free ends of the scaffold panel are joined to form the ring before the scaffold panel has been released from the elongated carrier region of the delivery catheter, but in other instances the first and second free ends of the scaffold panel may be joined after release of at least one of the free ends from the delivery catheter.

While the present application describes the use of the delivery system according to embodiments of the present invention with respect to the mitral valve, however, the delivery system is not limited to mitral valve replacement and may be used in any portion of the heart, vasculature, the cardiovascular system, or organ of the human or animal.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in order to provide what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.
FIG. 1A is a schematic illustration of a heart showing the flow pattern therethrough and location for delivery of a prosthetic heart valve;
FIGS. 1B-1D are schematic, cross-sectional illustrations of the heart showing various state of the art trans-catheter approaches that allow a practitioner to reach the treatment area, namely, a native mitral valve. FIG. 1B illustrates an antegrade approach wherein access is gained through the inter-atrial septum (IAS) maintained by the placement of a guide catheter over a guidewire. FIG. 1C illustrates a retrograde approach to the native mitral valve. FIG. 1D illustrates a trans-apical approach to the mitral valve using a trans-apical puncture;
FIGS. 2A-2B shows a schematic non-limiting illustration of the prosthetic device and/or support structure configured to be delivered with the system according to embodiments of the present invention;
FIG. 3 shows a schematic box diagram illustrating the delivery system according to embodiments of the present invention;
FIGS. 4A-4D show schematic non-limiting illustrative views of optional embodiments of the delivery system according to embodiments of the present invention; FIG. 4A shows the delivery system in use during delivery of a prosthetic device; FIG. 4B shows the delivery system with cover transparent to reveal the internal configuration; FIG. 4C shows the delivery system with cover removed so as to reveal the crimped prosthetic device in its delivery configuration; FIG. 4D shows a close up view of the distal end of the delivery system with the prosthetic device removed;
FIGS. 5A-5B shows a schematic non-limiting illustrative view of the delivery system during deployment of the prosthetic device according to embodiments of the present invention; FIG. 5A shows an optional embodiment for revealing and initiating deployment of the prosthetic device through the distal end of the delivery system; FIG. 5B shows an illustration of the prosthetic device during the stages of its delivery in the implantation site just prior to final deployment;
FIGS. 6A-6C show different view of a schematic non-limiting illustration of the prosthetic device in use with the delivery system just prior to deployment according to embodiments of the present invention; FIG. 6A shows a perspective view; FIG. 6B is a close up view revealing details of the distal end and coupling of the delivery system and prosthetic device; FIG. 6C is a further close up view revealing the coupling of the delivery system and prosthetic device according to embodiments of the present invention; ;
FIGS. 7A-7B shows a close-up view of a schematic non-limiting illustration of the coupling members of the prosthetic device and/or support structure and the delivery system according to embodiments of the present invention; FIG. 7A shows the proximal coupling members; FIG. 7B showing the distal coupling member;
FIGS. 8A-8B show close-up views of a schematic non-limiting illustration of the second coupling members according to embodiments of the present invention; FIG. 8A showing a side view; FIG. 8B showing a perspective view;
FIGS. 9A-9B show close up views of a schematic non-limiting illustration of the first coupling members; FIG. 9A showing a side view; FIG. 9B showing a perspective view; and
FIGS. 10A-10E show schematic non-limiting illustrations of an optional coupling members according to embodiments of the present invention; FIG. 10A is a perspective view of a closed coupling member; FIG. 10B shows a perspective view of an open coupling member; FIG. 10C shows a perspective view of an intermediate phase of coupling member; FIG. 10D shows a perspective view of the open coupling member; FIG. 10E shows a close-up view of the opened coupling member.
FIGS. 11A and 11B are top, isometric views of a presently preferred embodiment of an intraluminal support structure according to the present invention as claimed. FIG. 12A shows the scaffold panel of the intraluminal support structure in the absence of a valve structure. FIG.12B shows the scaffold panel with an attached valve structure.
FIGS. 12A and 12B are bottom, isometric views of the intraluminal support structure of FIGS. 11A and 11B, respectively.
FIGS. 13A and 13B are bottom, isometric views of the intraluminal support structure of FIGS. 11A and 11B, respectively, shown with the scaffold panel partially open.
FIG. 14 is a side view of the scaffold panel of the intraluminal scaffold structure embodiment of FIGS. 11A and 11B, 12A and 12B, and 13A and 13B, shown in a flattened configuration.
FIG. 14A is a side view of an alternative embodiment of a scaffold panel of the intraluminal scaffold structure of the present invention similar to that illustrated in FIG. 14 but having a spiral spring component for increasing flexibility when the scaffold panel is twisted for delivery.

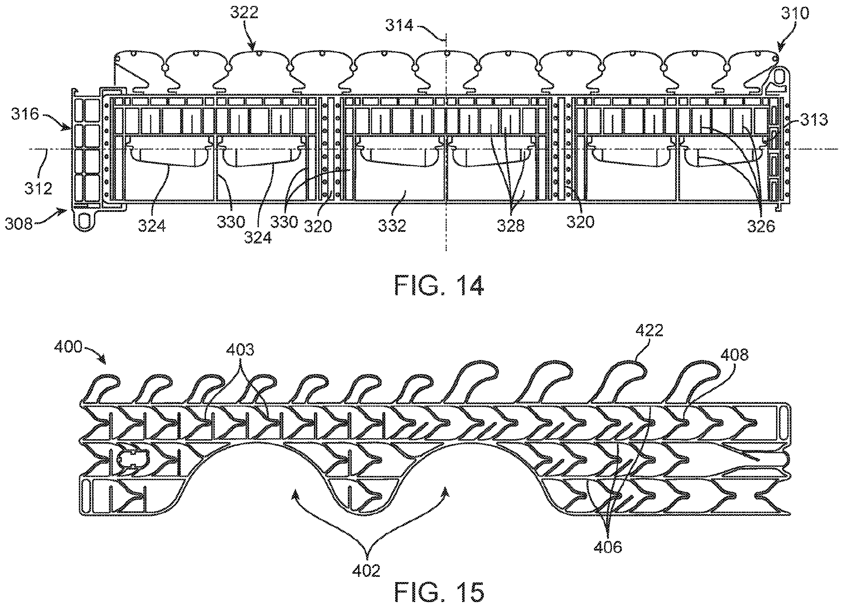

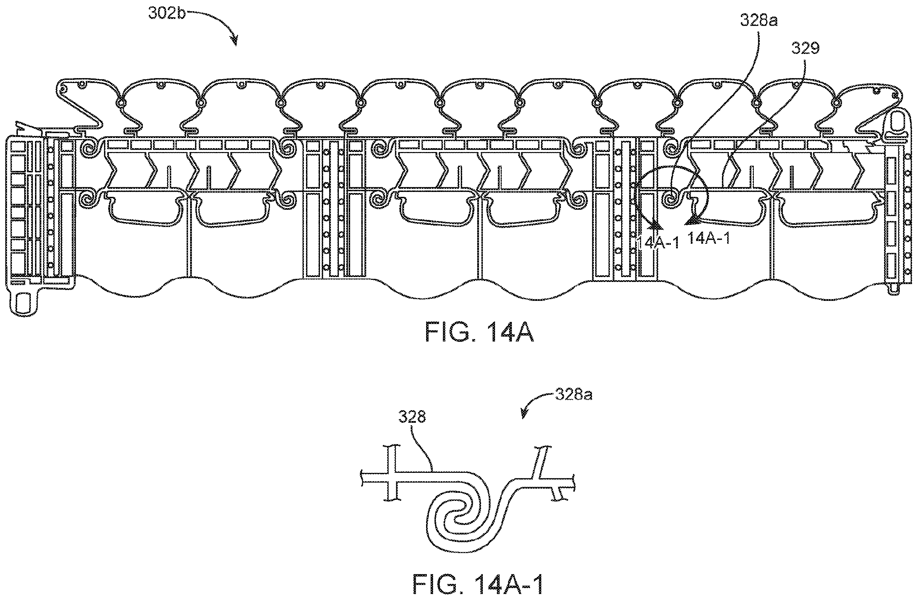

FIG. 14A-1 is a detailed view taken along line 14A-1:14A-1 in FIG. 14A.

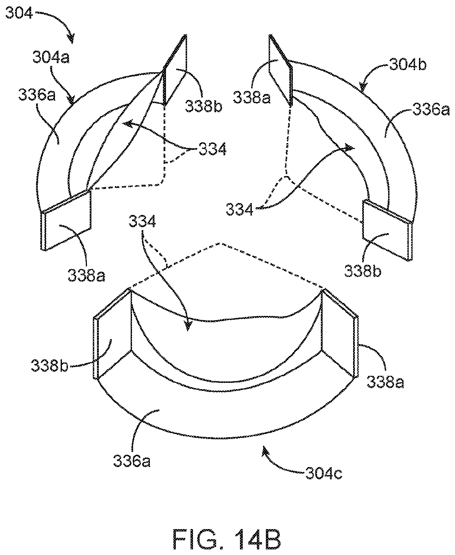

FIG. 14B is an exploded view of a valve structure including three valve leaflets, skirts, and fins in accordance with the principles of the present invention.
FIG. 15 is a side view of an alternative embodiment of a scaffold panel of the intraluminal scaffold structure of the present invention shown in a flattened configuration.

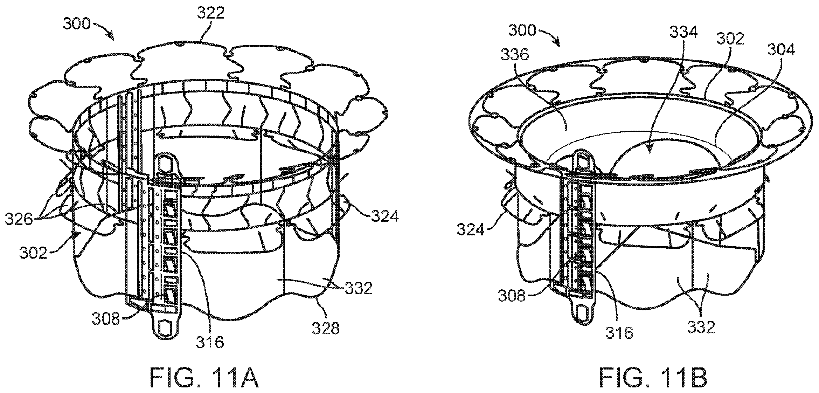

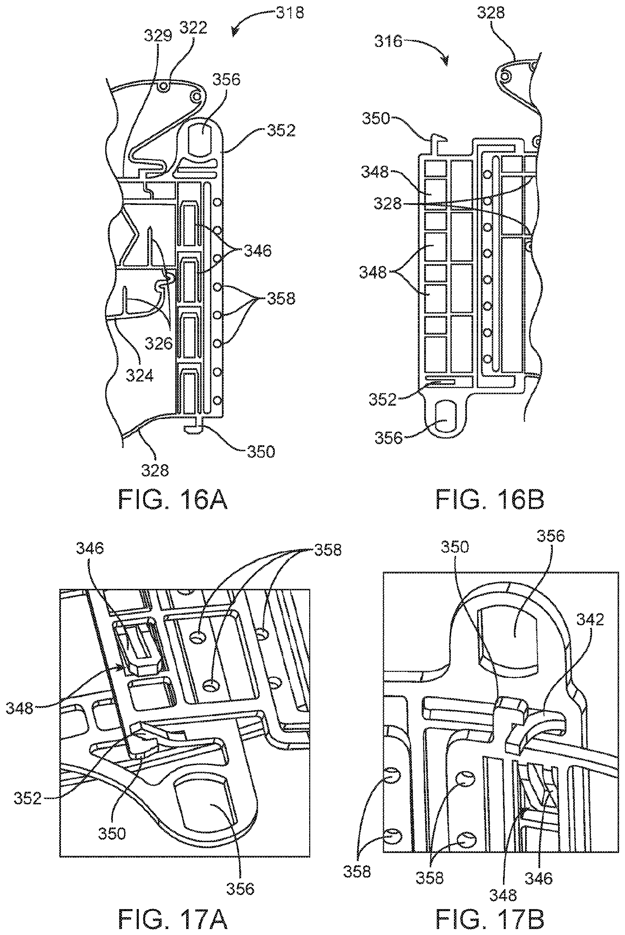

FIGS. 16A and 16B are detailed views of a first free and a second free end the scaffold panel of the intraluminal scaffold structure embodiment of FIGS. 11A and 11B, 12A and 12B, and 13A and 13B, showing a first coupling structure (FIG. 16B) and a second coupling structure (FIG. 16A) configured to be joined together to form the scaffold panel into a ring, according to the present invention as claimed.
FIGS. 17A and 17B are detailed views of the coupling structures of FIGS. 16A and 16B showing how the structures are coupled together to join the flat scaffold panel into a ring, according to the present invention as claimed.

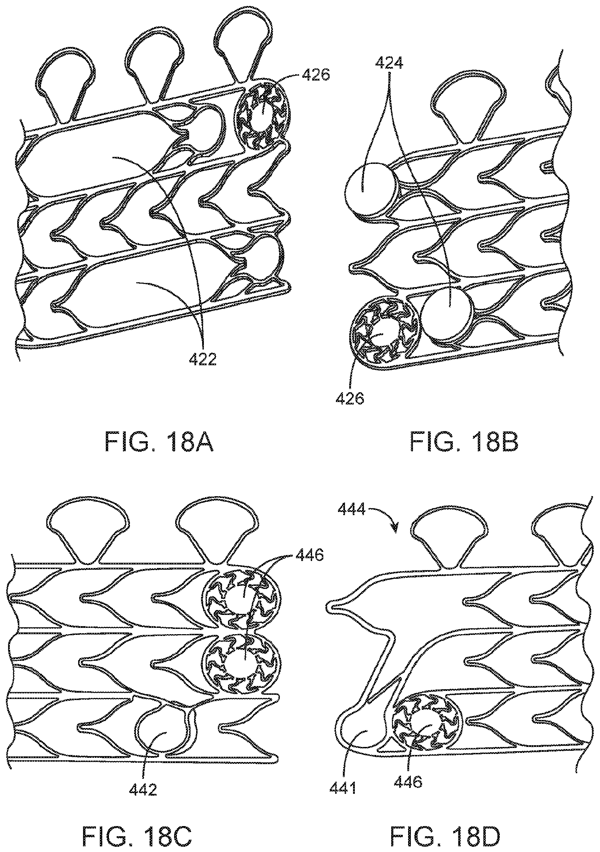

FIGS. 18A and 18B are detailed views of a first alternative coupling structure of the present invention.
FIGS. 18C and 18D are detailed views of a second alternative coupling structure of the present invention.

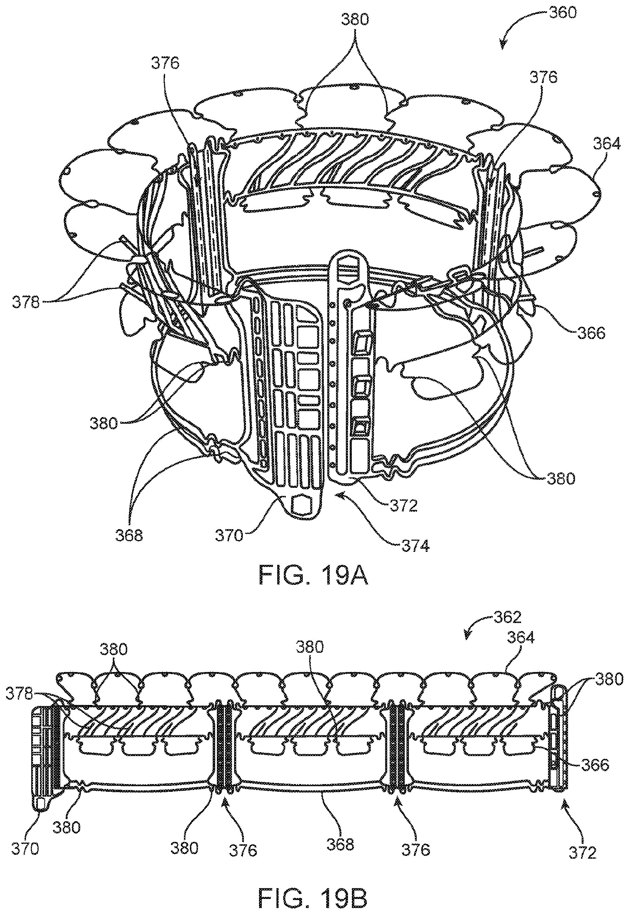

FIGS. 19A and 19B are isometric and rolled out views of an alternative embodiment of an intraluminal support structure constructed in accordance with the principles of the present invention, shown without a valve leaflet structure.

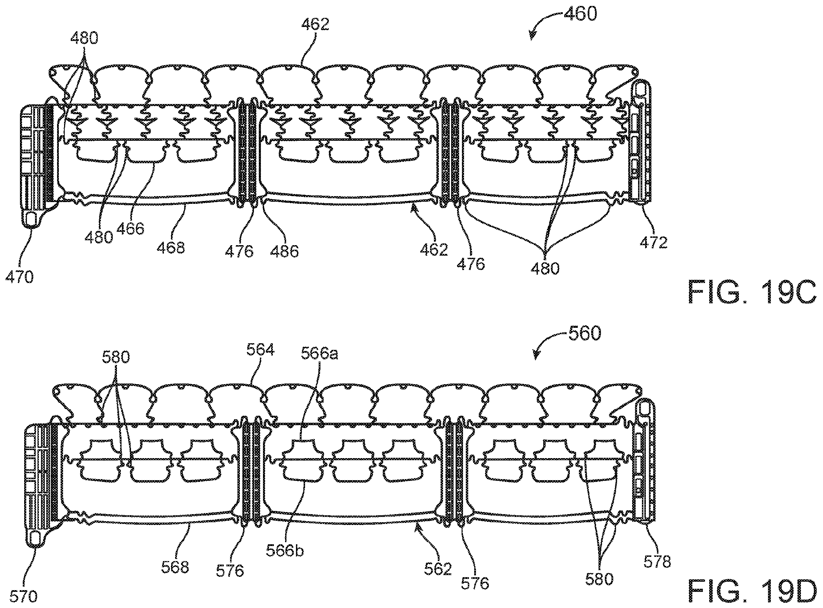

FIG. 19C is a rolled-out view of a further alternative embodiment of an intraluminal support structure in constructed in accordance with the principles of the present invention, shown without a valve leaflet structure.
FIG. 19D is a rolled-out view of a further alternative embodiment of an intraluminal support structure constructed in accordance with the principles of the present invention, shown without a valve leaflet structure.

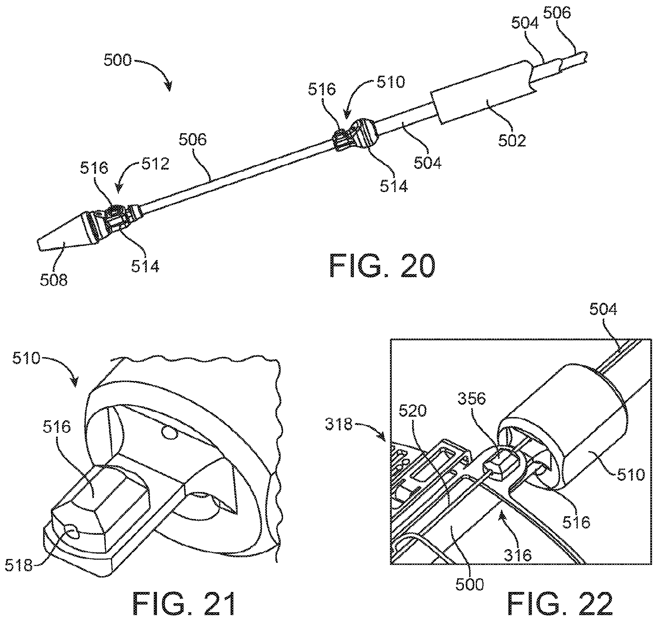

FIG. 20 is an isometric view of a distal region of a delivery catheter constructed in accordance with the principles of the present invention showing a elongate carrier region for delivery of the intraluminal support scaffold of the present invention to a target location in a patient's vasculature.
FIG. 21 is a detailed view of a proximal coupler on the delivery catheter for removably attaching the first coupling region of the intraluminal support scaffold when carried by the delivery catheter.
FIG. 22 is a detailed view showing the proximal coupler of FIG. 21 attached to the first coupling region of the intraluminal support scaffold.

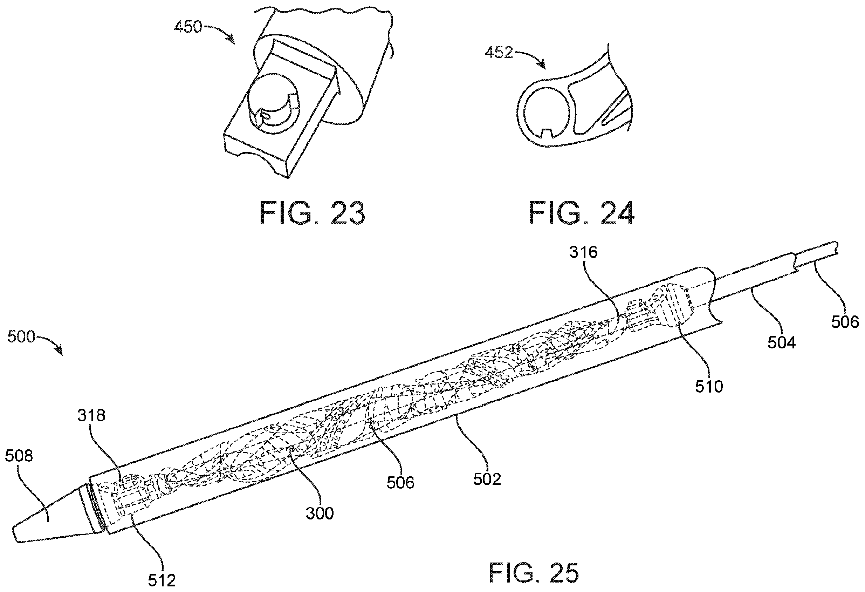

FIGS. 23 and 24 illustrates an alternative coupling mechanism for the detachably securing the intraluminal support scaffold of present invention to a delivery catheter.
FIG. 25 makes the intraluminal support scaffold of the present invention mounted on the distal region of the delivery catheter of the present invention in a configuration suitable for advancement in the patient's vasculature.

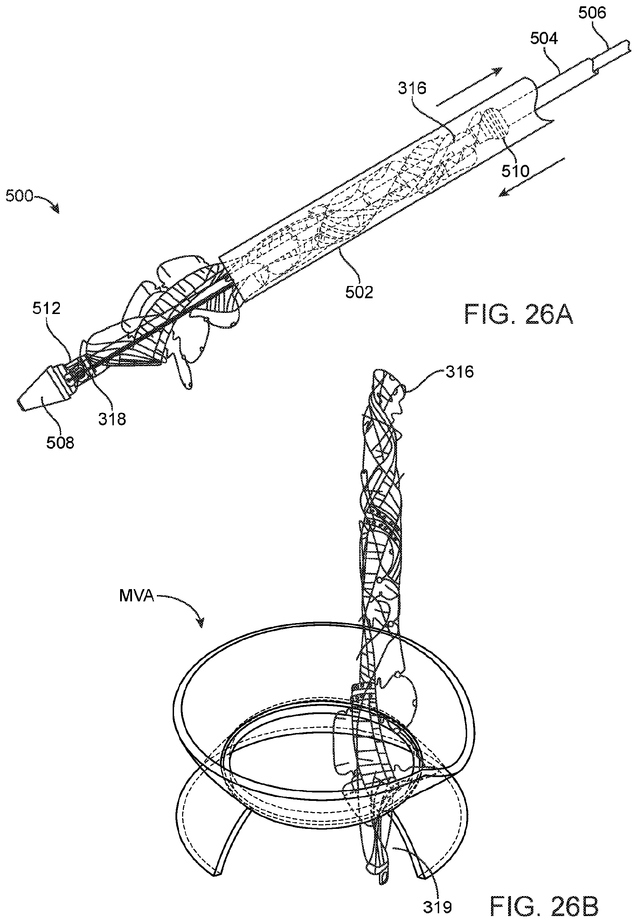

FIGS. 26A and 26B illustrate a first step in release of the intraluminal support scaffold from the delivery catheter. A sheath of the delivery catheter in FIG. 26A is shown in a transparent view to reveal a portion of the intraluminal support scaffold which is covered by the sheath. The intraluminal support scaffold in FIG. 26B is shown without the delivery catheter so that the configuration of the scaffold can be more clearly seen.

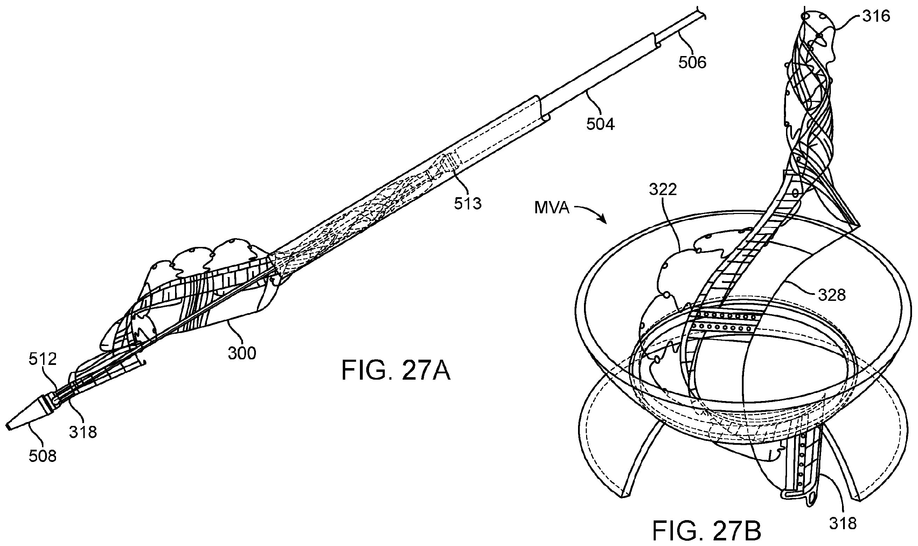

FIGS. 27A and 27B are views similar to FIGS. 26A and 26B illustrating a further step in release of the intraluminal support scaffold from the delivery catheter.

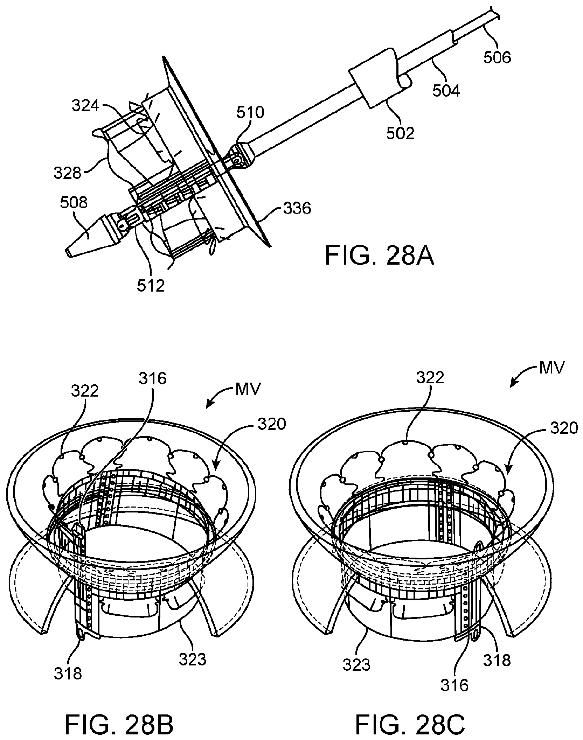

FIGS. 28A to 28C illustrate the final release of the intraluminal support scaffold from the delivery catheter. FIGS. 28B and 28C show the final joining of the first coupling structure to the second coupling structure to join the first and second together form the final ring structure.

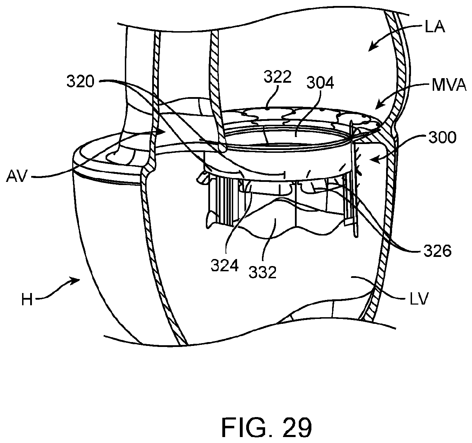

FIG 29 illustrates the intraluminal support structure comprising a prosthetic mitral valve in place in a patient's mitral valve after having been delivered in accordance with the principles of the present invention.

### DESCRIPTION OF THE PREFERRED EMBODIMENTS

The present invention generally relates to trans-catheter delivery systems used to deliver medical implants into remote location of a human body and in particular, to such a delivery system for delivering medical implants to a human heart. Particularly embodiments of the present invention provide a catheter-based delivery system configured to deliver and deploy a prosthetic mitral valve.

Embodiments of the present invention provide a delivery system configured to deliver a prosthetic mitral valve in a crimped-planar configuration. Such a delivery system is adept to delivery in a manner that is best suited to properly fit and/or conform to annulus of the native mitral valve.

The delivery system of the present invention provides for delivery of a planar-crimped prosthetic device for example in the form of a valve, in that the prosthetic valve support structure is delivered in a planar manner as it disassociates from the delivery system.

The principles and operation of the present invention may be better understood with reference to the drawings and the accompanying description.

Now referring to FIGS. 1A-D showing a heart and the prior art delivery systems used to reach the heart in various known minimally invasive approaches as depicted in FIGS. 1B-D. Such prior art delivery systems are typically limited in that the system is configured to delivery and deploy prosthetic devices via the distal end and/or distal tip of the delivery catheter. The limitations of such systems are realized when the prosthetic device is radially crimped during delivery, making it difficult to maneuver during the approach to the implantation site, heart, as the prosthetic device is next as flexible during the process. Such radial crimping requires radially expanding the prosthetic device during deployment, so as to allow the prosthetic device, stent and/or valve, to assume its final (in use) configuration. The steps necessary to expand the prosthetic device further limit the procedure.

Embodiments of the present invention provide a delivery system 200 that overcomes the limitations of the prior art in that the system 200 is configured for use with a utilizes a planar crimped prosthetic device 50, that is readily flexible and maneuverable during delivery. Furthermore, delivery system 200 further allows for improved deployment of the prosthetic device in the implantation site as the deployment does not require radial expansion.

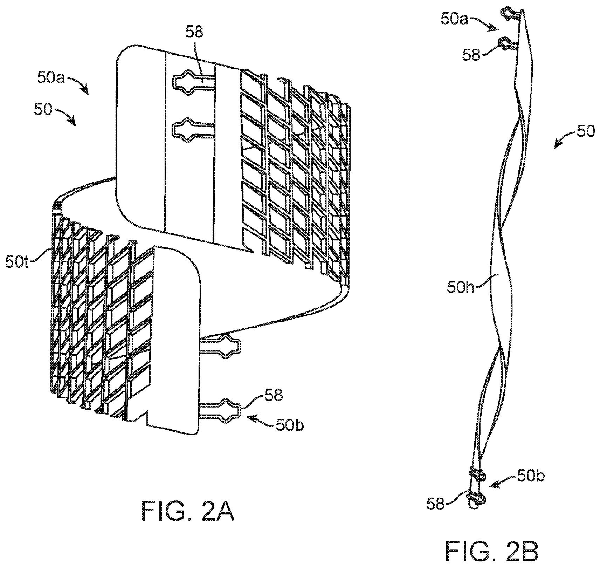

FIGS. 2A-B show a schematic illustration of a prosthetic device 50 that is delivered to an implantation site with the system 200 according to embodiments of the present invention. Prosthetic device 50, for example including but not limited to a vessel support structure and/or a prosthetic valve, stent or the like, that may be delivered with the delivery system 200 according to the present invention is characterized in that it is a planar crimped prosthetic device 50 during delivery. That is, while prior art delivery system are configured for the delivery of radially crimped tubular (non-planar) prosthetic devices, embodiments of the present invention provide a delivery system for delivery a planar (non-tubular) prosthetic device 50. FIG. 2A shows, a prosthetic device 50 that may be delivered with system 200 according to embodiments of the present invention. Prosthetic device 50 is a planar, non-tubular, structure having two open ends 50a,50b wherein each end features at least one or more coupling member 58.

Prosthetic device 50 may form its final and/or deployment configuration of the support structure (not shown) for example including but not limited to a stent, vessel support structure, valve or the like, when both ends 50a, 50b are closed with one another over said at least one or more coupling members 58. Preferably the deployment configuration (not shown) is formed at the implantation site during deployment from delivery system 200, according to embodiments of the present invention.

FIG. 2A shows an intermediate and/or transitional state 50t of prosthetic device 50 while it is associated with delivery system 200 and just prior to when the prosthetic device is closed about its ends 50a, 50b over the coupling member 58. Accordingly, FIG.2A shows a transitional crimped planar configuration of prosthetic device 50, near the end of the delivery procedure so as to recover its working (in use) configuration with the prosthetic device.

FIG. 2B shows prosthetic device 50 in its fully crimped-planar helical configuration 50h that is utilized at the onset of the delivery process with delivery system 200. In embodiments when system 200 is loaded with the prosthetic device 50 and/or a support structure ready for the onset of delivery it is preferably assumed in the helical crimped-planar configuration 50h, where most preferably such a helical crimped-planar configuration allows delivery system 200 the required flexibility and maneuverability within the vasculature leading to the implantation site and/or delivery site.

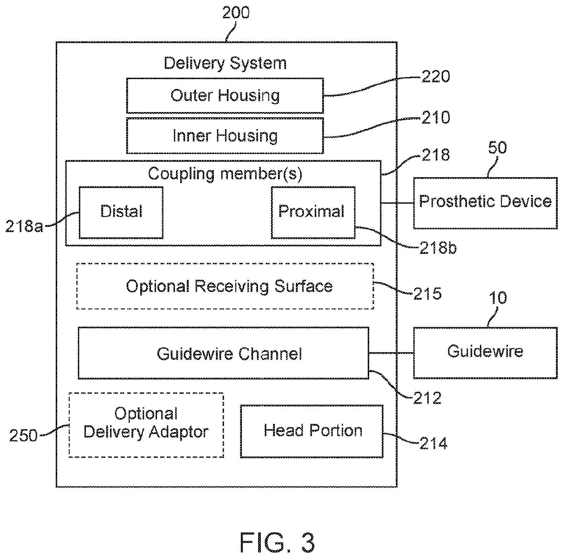

FIG. 3 shows a schematic box diagram depicting the trans-catheter delivery system 200 according to embodiments of the present invention. Delivery system 200 is a trans catheter delivery system that utilizes a guidewire 10 as a means for reaching the remote anatomical location for the delivery of the prosthetic device. The guidewire is introduced in a manner as is known in the art.

The delivery system 200 comprises an inner housing 210, interchangeably referred to as device housing member, and an outer housing 220, interchangeably referred to as cover member. Delivery system 200 is maneuvered to the implantation site along the guidewire 10.

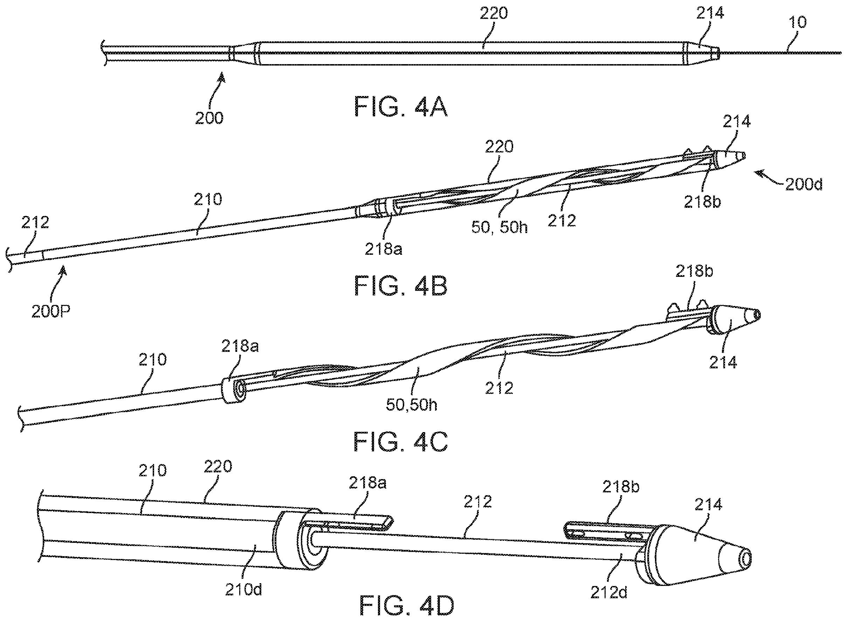

Inner housing 210 is preferably a cylindrical body comprising an internal guidewire channel 212. Guidewire channel 212 is preferably configured to receive and/or associate with a guidewire 10, FIG. 4A. Preferably guidewire channel 212 is an independent channel that is concentric with inner housing 210 allowing system 200 to be advanced along guidewire 10 in a concentric manner.

In embodiments, guidewire channel 212 comprises a distal end 212d (FIG. 4D) that is associated with a head portion 214. Head portion 214 facilitates maneuvering and guiding of system within the vasculature. Head portion 214 features a central internal guidewire lumen that is in fluid communication and/or continuous with internal guidewire channel 212. Preferably head portion 214 is an atraumatic and compliant member allowing for maneuverability and safety, such that it does not damage the surrounding and/or host tissue. In embodiments head portion 214 is configured so at to prevent damaging any of the cardiac tissue and in particular the left ventricle.

In embodiments inner housing 210 comprises at least a portion of coupling module 218 configured for coupling with and/or securing prosthetic device 50 with delivery system 200. In embodiments, distal end of inner housing 210d, FIG. 4D, is configured to couple with and/or receive a portion of coupling module 218, more preferably a first coupling member 218a.

Preferably coupling module 218 is configured to associate with and/or couple and/or receive corresponding coupling members provided on prosthetic device 50, for example coupling members 58 featured on prosthetic device 50, a non-limiting example of which is schematically depicted in FIGS. 2A-B.

In embodiments coupling module 218 preferably features at least two or more coupling members 218a,218b, that are configured to associate and/or retain at least a portion of prosthetic device 50, more preferably about an end thereof 50a,50b. Optionally coupling module 218 may comprise additional coupling members (not shown) configured for associating and/or supporting prosthetic device 50 along a body portion thereof, not shown.

Coupling module 218 preferably features a first coupling member 218a that is disposed about a distal portion of inner housing 210, and a second coupling member 218b, that is disposed about a distal portion of guidewire channel 212.

In embodiments first coupling member 218a is preferably configured to couple with a first end 50a of prosthetic device 50 while second coupling member 218b is configured to be coupled with a second end 50b of prosthetic device 50.

In embodiments first coupling member 218a is configured to be coupled and/or secured with a portion of inner housing 210, preferably distal end 210d, FIG. 4D. More preferably, a portion of first coupling member 218a is secured and/or affixed with an external surface of an end of inner housing 210.

In embodiments second coupling member 218b is configured to be coupled and/or secured with a portion of head portion 214. Most preferably, a portion of second coupling member 218b is secured and/or affixed with a proximal end of head portion 214.

In embodiments at least a portion of coupling members 218a, 218b are configured to be associated with a portion of guidewire channel 212.

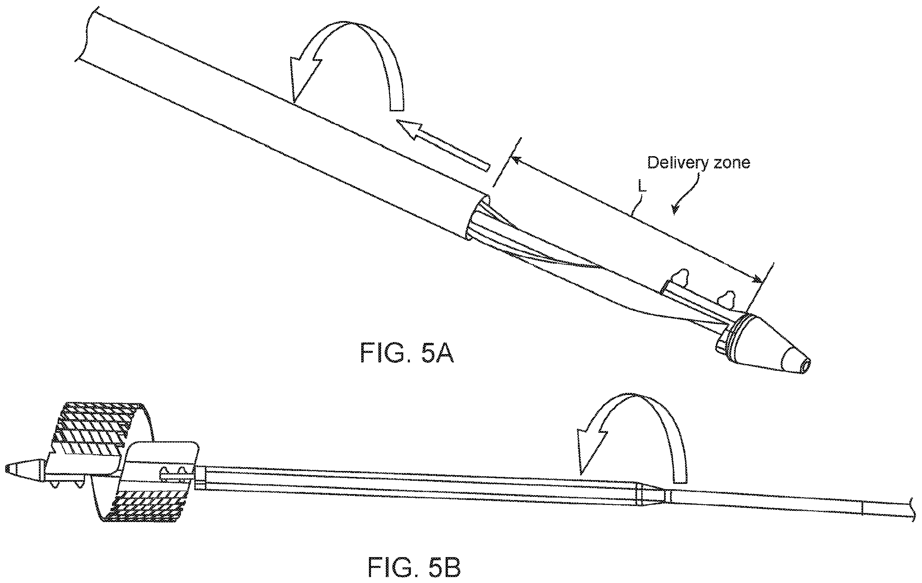

Outer housing 220 is preferably a substantially cylindrical tubular housing having an open central channel configured for receiving and/or housing inner housing 210, and guidewire channel 212, coupling module 218 and prosthetic device 50. Most preferably, during the deployment procedure outer housing 220 may be maneuverable over internal housing 210, so as to reveal the contents of delivery system 200 that are preferably associated with coupling module 218 and preferably between at least two coupling members 218a and 218b. Most preferably prosthetic device 50 is disposed within the internal lumen of outer housing 220. along an external surface of guidewire channel 212, wherein guidewire channel 212 forms a central axis of the prosthetic device 50. In embodiments, outer housing 220 may be moved over guidewire channel 212 and inner housing 210 by a distance 'L', FIG. 5A, wherein distance 'L' is equivalent to at least one dimension, preferably equivalent to the height of the prosthetic device 50, in its deployment configuration, for example as shown in FIG. 2A.

In an optional embodiment guidewire channel 212 may further feature a prosthesis receiving surface 215 that may be configured to associate with, receive and/or retain at least a portion of prosthetic device 50. Optionally, receiving surface 215 may be configured to facilitate housing and/or securing at least a portion of prosthetic device 50 in a crimped-planar configuration, for example in a helical crimped-planar configuration 50h, along the body of the prosthetic device rather than either of ends 50a, 50b, as shown in FIG. 2B.

In an optional embodiment, receiving surface 215 may be configured to be a male receiving surface featuring a raised and/or external receiving surface, for example including but not limited to a receiving fin (not shown). In embodiments such a receiving fin may be configured to have a helical fin configuration, to accommodate helical crimped planar configuration 50h.

In an optional embodiment a helical fin configuration may be configured to have a constant pitch along the length of the receiving surface. In some embodiments a helical fin configuration may be configured to have variable pitch along the length of the receiving surface.

In an optional embodiment the receiving surface 215 may be configured to be a female receiving surface featuring a recessed receiving surface and/or a grooved receiving surface. In such optional embodiment, a female receiving surface may be configured to have a helical groove and/or recess along at least a portion of the external surface of guidewire channel 212.

In an optional embodiment a helical groove configuration may be configured to have a constant pitch along the length of the receiving surface. In some embodiments a helical groove configuration may be configured to have variable pitch along the length of the receiving surface.

In an optional embodiment delivery system 200 may further comprise an optional movement and/or delivery adaptor 250 that may be utilized with the inner housing 210 and/or guidewire lumen 212 and/or coupling module 218 to facilitate delivery and/or deployment of the prosthetic device 50. Optionally, delivery adaptor 250 may be configured to facilitate unfurling of prosthetic device 50 so as to facilitate disassociating and/or release it from coupling module 218, Optionally delivery adaptor 250 may be provided in optional form for example including but not limited to at least one or more selected from: a linear to rotational movement adaptor, translational movement adaptor, linear displacement adaptor, movement of adaptor, rotational adaptor, helical movement adaptor, counter-clockwise direction adaptor, clockwise direction adaptor, (CW), lateral movement adaptor, the like or any combination thereof.

In some embodiments delivery adaptor 250 may be provided to facilitate transitioning prosthetic device 50 from one state to another by providing at least one or more stimuli for example including but not limited to temperature change, electromagnetic field, electric current, temperature increase, temperature decrease, light, photoelectric field, acoustic energy, frequency, the like or any combination thereof.

In embodiments optional delivery adaptor 250 may be provided to facilitate the advancement of prosthetic device 50 through the delivery system 200 and to further facilitate deployment of prosthetic device 50 into the delivery site. In order to facilitate such delivery adaptor 250 may optionally be fit with a low friction member, for example including but not limited to a bearing or a ball bearing ring or the like friction minimizing device.

FIGS. 4A-D, 5A-B, 6A-C, 7A-B, 8A-B and 9A-B collectively show a non-limiting schematic illustrative depiction of delivery system 200 according to an embodiment of the present invention. FIG. 4A shows a schematic illustrative side view of a delivery system 200 according to embodiments of the present invention. As previously described system 200 comprises an outer housing 220 that encompasses an inner housing 210, that features a prosthetic device 50 configured for delivery in a crimped planar (non-tubular) configuration. Outer housing 220 is configured to receive and house inner housing 210, as best seen in FIG. 4B. Inner housing 210 is configured to associate with a prosthetic device 50, for example including but not limited to a prosthetic device and/or a vessel support structure, and/or a prosthetic valve, stent or the like, FIG. 4B. Preferably the prosthetic device 50 is associated with delivery system 200 via coupling module 218 featuring a first coupling member 218a and a second coupling member 218b, as best seen in FIG. 4D.

In embodiments, the prosthetic device 50 may be indirectly associated with a portion of inner housing 210, via coupling module 218, in a planar-crimped helical configuration 50h, as shown in FIGS. 4B-C.

In embodiments the delivery system 200 is characterized in that deployment of prosthetic device 50 is provided at a distal end 200d at head portion 214, FIGS. 5-6.

In embodiments system 200 is delivered to a treatment site, for example including but not limited to the heart, via a guidewire 10, FIG. 4A that is preferably disposed centrally within a guidewire channel 212 disposed intraluminal with inner housing 210 and outer housing 220, for example shown in FIGS. 4A-B.

FIG. 4B shows an embodiment of delivery system 200 where outer housing 220 is rendered transparent so as to show the components disposed therein. Accordingly, outer housing 220 is configured to be a sheath for housing additional components of delivery system 200.

FIG. 4C shows a close-up perspective view of the distal end of delivery system 200 where outer housing 220 has been removed to reveal the additional components and their arrangement therein. As shown guidewire channel 212 is internal to inner housing 210, each configured to associate with a portion of coupling module 218. As shown, helical crimped-planar configuration 50h of prosthesis 50 is disposed about an axis formed by guidewire channel 212, and wherein each end of prosthesis 50 is coupled and/or associated with system 200 with coupling module 218 over a first coupling member 218a and a second coupling member 218b.

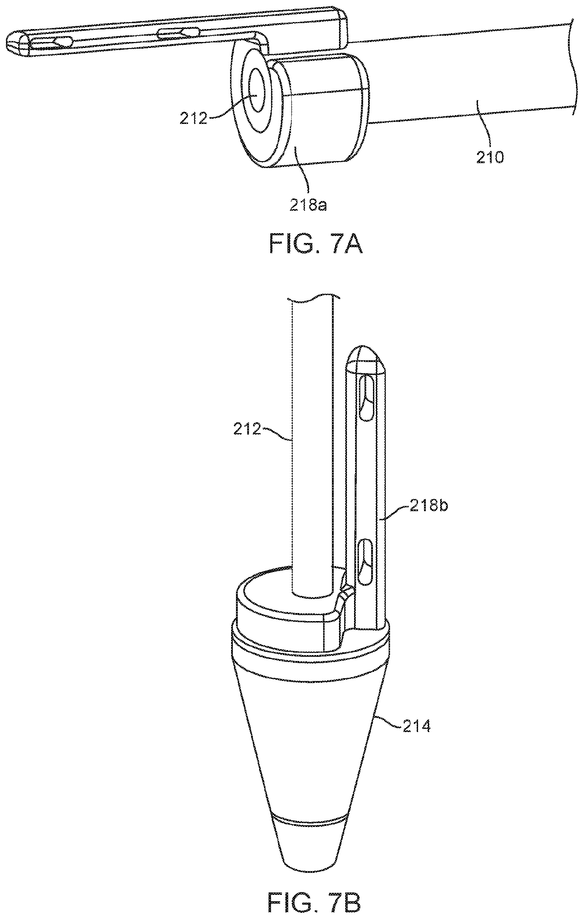

FIG. 4D provides a further close up view of coupling module 218 featuring first coupling member 218a and second coupling member 218b. As previously described first coupling member 218a is associated with inner housing 210 about a proximal end and with guidewire channel 212 about a distal end thereof, also shown in FIG. 7A. As previously described, second coupling member 218b is associated with guidewire channel 212 about a proximal end and with head portion 214 about a distal end thereof, for example as shown in FIG. 7B.

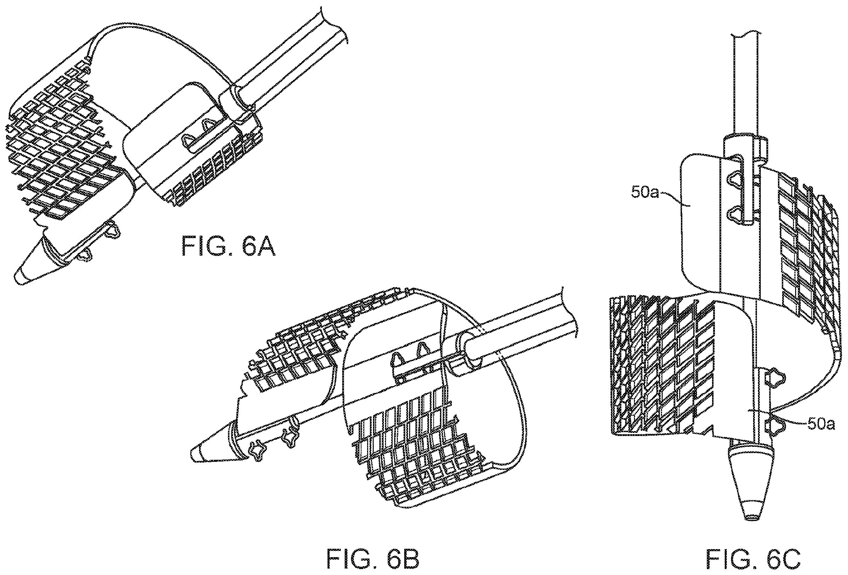

Now referring to FIGS. 5A-B and 6A-C that further show a delivery method according to embodiment of the present invention wherein prosthetic device 50 is deployed in a planar and/or helical crimped-planar configuration 50h and is delivered about distal end 200d near head portion 214. FIG. 5A shows how a prosthetic device 50 associated with delivery system 200 about coupling module 218, is deployed at a delivery site. In preferred embodiment, outer housing 220 is displaced proximally by a length 'L' so as to reveal a delivery zone and/or deployment area where prosthesis 50 will be deployed. Optionally inner housing 210 may be advanced and/or displaced distally in order to reveal the delivery zone. Once the delivery zone has been revealed first coupling member 218a and second coupling member 218b are advanced toward one another so as to de-crimp prosthetic device 50, for example as shown in FIG. 5B. In preferred embodiments, coupling module 218 is configured to release prosthetic device 50 by having one coupling member 218a or 218b move toward the other while the other coupling member remains stationary. For example, first coupling member 218a may be advanced along guidewire channel 212 while second coupling member 218b remains stationary adjacent to head portion 214 at the delivery site. In embodiments first coupling member 218a and second coupling member 218b may be advanced toward one another by way optional movements for example including but not limited to linear movement, rotational movement, or the like. In an optional embodiment a delivery module 250 may be utilized to advance and/or facilitate de- crimping of prosthesis 50.

In embodiments, most preferably advancement of coupling members 218a, 218b toward one another continues until, ends of 50a.50b of prosthesis 50 reach one another and are able to be released from delivery system 200. FIGS. 6A-C shows various views of the delivery process as coupling members 218a, 218b approach one another.

In embodiments, a prosthetic device 50 may be loaded onto delivery system 200 in a similar fashion as that described above wherein, an end 50a,50b of device 50 is associated with a corresponding coupling member 218a,218b over prosthesis coupling member 58. Once coupling members 218a, 218b are associated with respective coupling members 58, coupling members 218a, 218b are displaced away from one another, optionally with the assistance of a delivery module 250, to assume the helical crimped-planar configuration 50h, about guidewire channel 212.

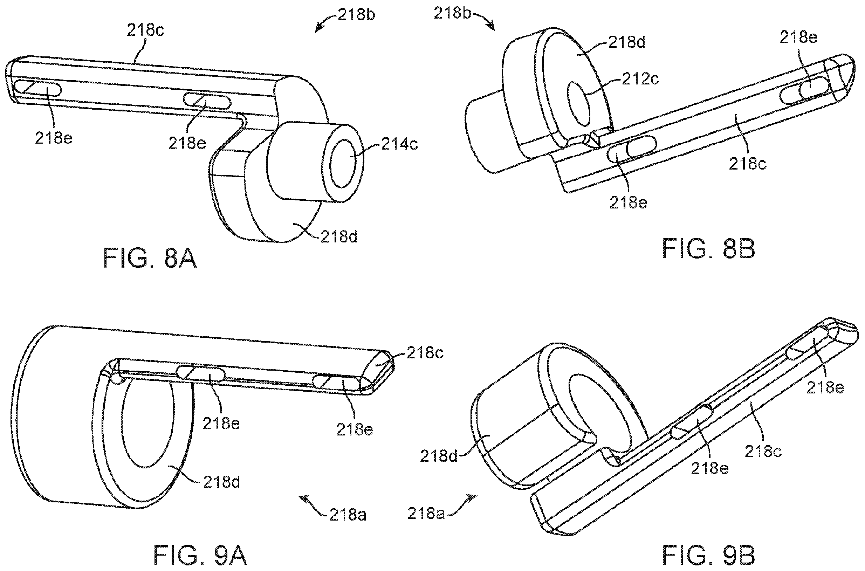

Now referring to FIGS. 8A-B, a close-up view of second coupling member 218b is configured to be associated with head portion 214, as shown in FIG. 7B. Coupling member 218b comprises a base 218d configured for coupling and/or interfacing between head portion 214c along a distal side of base 218d and guidewire channel 212c along a proximal side of base 218d.

Coupling member 218b further features a prosthesis coupling portion 218c comprising at least one or more coupling element 218e adapted for receiving and/or associating and/or securing with prosthesis coupling member 58.

In embodiments coupling portion 218c extends from base 218d and features at least one or more coupling elements 218e. In embodiment coupling elements 218e may be provided as a male or female coupling member and is not limited to the female coupling recess member shown here.

Now referring to FIGS. 9A-B that shows a close-up view of first coupling member 218a that is configured to be associated with inner housing 210, as shown in FIG. 7A. Coupling member 218a comprises a base 218d configured for coupling and/or interfacing between a portion of inner housing 210 along a proximal side of base 218d and guidewire channel 212 along a distal side of base 218d.

Coupling member 218a further features a prosthesis coupling portion 218c comprising at least one or more coupling element 218e adapted for receiving and/or associating and/or securing with prosthesis coupling member 58 (FIG. 2A).

In embodiments coupling portion 218c extends from base 218d and features at least one or more coupling elements 218e. In embodiment coupling elements 218e may be provided as a male or female coupling member and is not limited to the female coupling recess member shown here that may be coupled to the male coupling member 58 of prosthesis 50 (FIG. 2A).

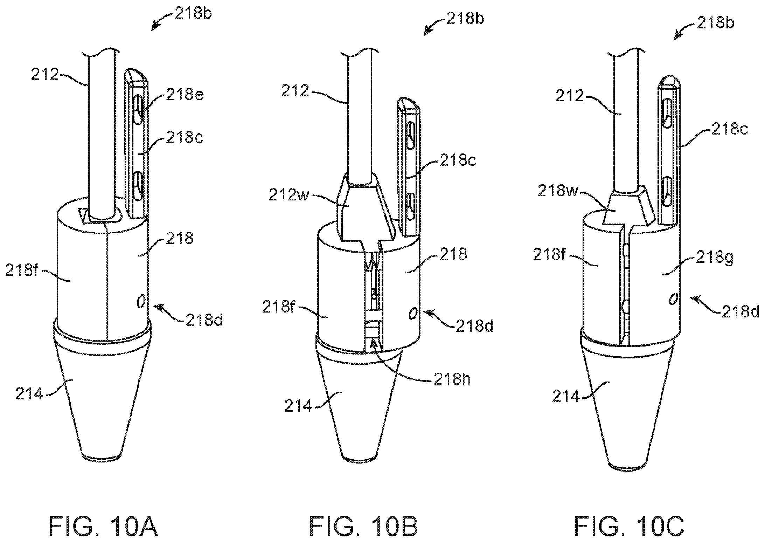

Now referring to FIGS. 10A-E that show a coupling member 218a, 218b of coupling module 218 that features a based 218d having an internal actuating member 218h according to an embodiment of the present invention. FIGS. 10 A-E show coupling member 218b, similar to that previously shown and describe, that further comprise an actuating member 218h that is disposed internal to base 218d. While the following description and drawings show actuating member 218h as implemented with second coupling member 218b, the present application is not limited to such an implementation as actuating member 218h may be similarly disposed with first coupling member 218a.

FIG. 10A shows second coupling member 218b having a base 218d that is formed from two portion a fixed member 218f and a moveable base member 218g wherein an actuating module 218h that is disposed internal to the base 218d between fixed portion 218f and moveable portion 218g.

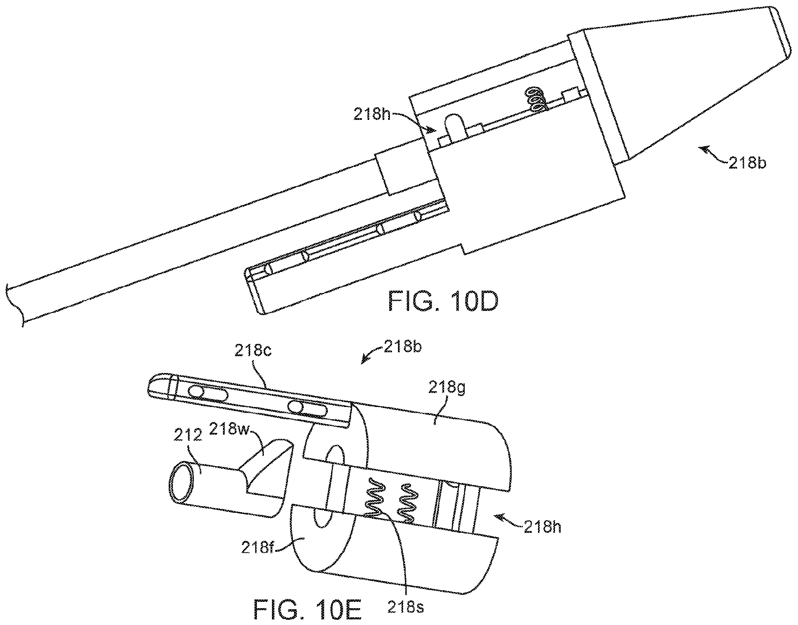

In embodiments, actuator module 218h provides for separating and/or opening base 218d such that fixed portion 218f and moveable portion 218g separate from one another so as to open base 218d, for example as shown in FIG. 10B and FIG. 10D. In embodiments, the separation of base 218d facilitates in loading and releasing prosthetic device 50 from coupling module 218.

In embodiments actuator module may be comprise optional actuators for example including but not limited to a spring under tension, recoil spring, linear spring, motor, magnet, electromagnet, gear, cam, shaft, the like or any combination thereof.

In embodiments, actuator module 218h may be configured to be actuated and/or controlled by mechanical movement for example including but not limited to linear movement, rotational movement, or a combination thereof.

FIG. 10E shows an exploded view of actuator module 218h showing its components comprising wedge actuator 218w and spring members 218s. In embodiments wedge actuator 218w is provided with a corresponding rail, preferably along moveable base portion 218g, so as to urge base portion 218g to separate from fixed base portion by a preset distance that is determined by spring member(s) 218s. Most preferably spring member(s) 218s provides the necessary tension to open and close base 218d. FIG. 10A shows the closed configuration base 218d while FIG. 10B shows the open configuration wherein wedge 218w has been moved proximally to open base. FIG. 10C shows the closure of base 218d wherein wedge 218w is urged distally to close base 218d.

In embodiments wedge actuator 218w is directly and/or indirectly associated with a portion of guidewire channel 212.

Accordingly, embodiments of the present invention provide for use of a single delivery system irrespective of the final diameter and/or size of the support structure being delivered this is specifically due to the crimping along the short axis as previously described.

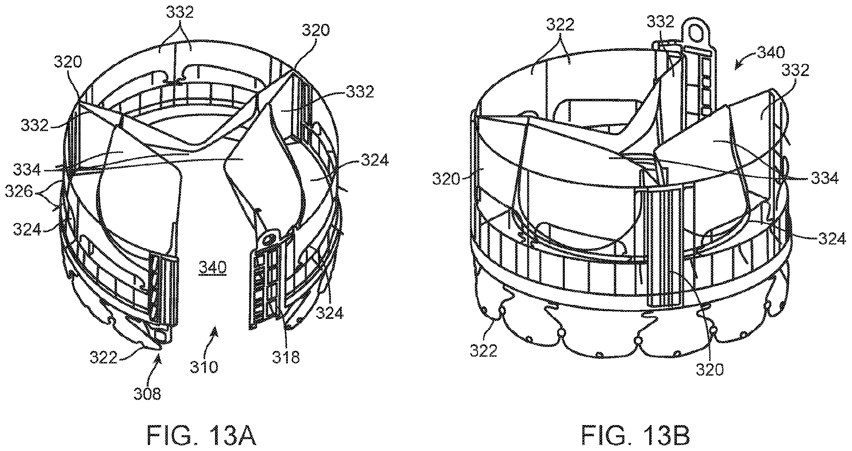

Referring now to FIGS. 11A to 13B and 14, a presently preferred design of an intraluminal support structure 300 constructed according to the present invention as claimed will be described. The intraluminal support structure 300 comprises a scaffold panel 302 having both a flat configuration, as seen in FIG. 14, and a ring or circular configuration, as best seen in FIGS. 11A to 12B. A partially closed structure is shown in FIGS. 13A and 13B where a gap 340 remains between free ends 308 and 310 of the scaffold panel 302. Typically, the scaffold panel 302 will be attached to a valve structure 304. For convenience, the scaffold panel 302 is shown without the valve structure 304 in FIGS. 11A, 12A, and 14, while the combination of the scaffold panel and the valve structure is shown in FIGS. 11B, 12B, 13A, and 13B.

The scaffold panel 302 when in a flattened configuration, as shown in FIG. 14, has a first free end 308, a second free end 310, a longitudinal axis 312, and a transverse axis 314. When the scaffold panel is formed into a ring or other circular structure, as seen in FIGS. 11A to 12B, the free ends 308 and 310 are joined by first and second coupling structures 316 and 318, and the longitudinal axis 312 becomes a circumferential line.

The intraluminal support structure 300 is shown in a top view (as would be seen from the left atrium when implanted in a mitral valve annulus) in FIGS. 11A and 11B and has an upper crown structure 322 and a lower crown structure 324 circumscribing the circularized scaffold panel 302. While the two crown structures 322 and 324 are illustrated as lobed or segmented flanges which extend radially outwardly from the circularized scaffold panel 302, they may have any other structure suitable for their purpose which is to capture a valve annulus or similar anatomy when the intraluminal support structure is deployed. Thus, the crown structures 322 and 324 may have any one or more of a variety of specific constructions and may include various anchoring features such as barbs 326, hooks, pins, or other elements which help secure or immobilize the intraluminal support structure when implanted.

When joined together, as shown in FIGS. 11A to 12B, the first and second coupling structures 316 and 318 will form a first of a plurality of "commissural posts," i.e., structures to which the commissures of the valve structure 304 will be attached, as described in greater detail below. For tricuspid valves, the scaffold panel 302 typically includes two additional commissural posts 320 which are typically permanent, fixed elements in the scaffold, i.e., they do not separate when the scaffold panel 302 is partially or fully opened, as shown in FIGS. 13A, 13B, and 14.

While a presently preferred scaffold pattern is shown in FIG. 14, it will be appreciated that many different specific scaffold patterns could be utilized. The pattern of FIG. 14 is typically formed by laser cutting of a flat or tubular "blank," but could alternatively be fabricated by photolithography or other known methods. The scaffold panel 302 may be formed from any one of a variety of materials, typically being formed from nickel-titanium alloy, stainless steel, or other metal alloy having a shape memory and being suitable for human implantation, and is characterized by a plurality of longitudinal members 328 and transverse members 330 which are typically formed into an orthogonal pattern so that the structure resists compression in both the longitudinal and transverse directions when in its flattened configuration. When in its circularized configuration, the intraluminal support structural 302 will further resist radial compression while allowing some degree of deformation in response to the forces applied by a beating heart. The scaffold panel 302 further includes a plurality of openings 332 to allow for free flow of blood when the intraluminal support structure 300 is implanted proximate a cardiac valve, as described in detail below.

It will be appreciated, however, that the scaffold panel of the present invention may have alternative patterns, some of which may be more compliant or compressible in the longitudinal and/or transverse directions. For example, as shown in FIG. 15, an alternative scaffold panel 400 comprises a plurality of longitudinal support members 406 typically joined by compressible links 408 which allow the panel to be compressed in a transverse direction while remaining relatively non-compressible in the longitudinal direction. The alternative scaffold panel 400 also has an alternative pattern for its upper crown and openings which allow free blood flow after implantation similar to those for openings 332 scaffold panel 302, as described above.

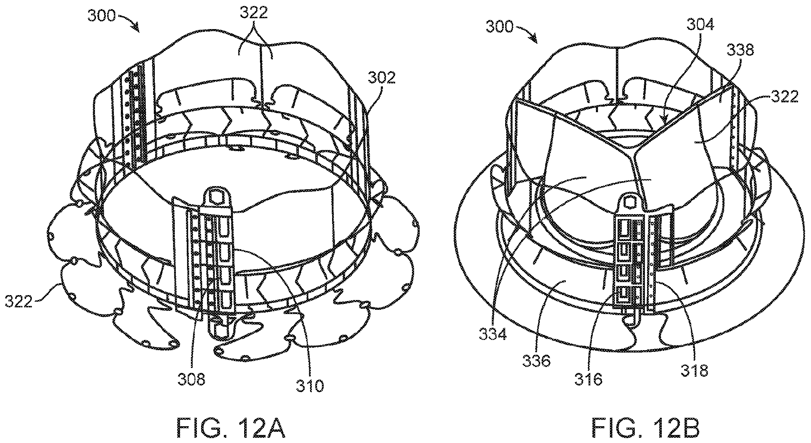

Attachment of the valve structure 304 to the scaffold panel 302 is best seen in FIGS. 11B, 12B, and 13B. Exemplary valve structures 304 include a plurality of leaflets 334, a skirt 336, and a plurality of fins 338. Each fin 338 extends radially outwardly from the valve structure 304 when the leaflets 334 are brought together as shown for example in FIG. 12B. The fins 338 thus allow the diameter of the leaflet structure to be smaller than the diameter of the scaffold panel after it has been circularized. This is advantageous as it decreases stress and strain on the leaflets and on their attachment to the scaffold as well as allowing the leaflet height to be reduced which in turn lessens potential interference with the with the left ventricular outflow tract when the intraluminal support structure is implanted in a mitral valve. The skirt 336 seals the resulting annular gap between the bottom on the circularized scaffold panel and the bottom of the valve structure 304. The design of the valve structure 304 further allows the valve structure to change configuration during the loading of the scaffold panel onto a delivery catheter and transitioning of the scaffold panel from a helically open configuration to a cylindrical closed configuration during release from the delivery panel and implantation. The segmented valve structure design also allows replacement valves to have a size nearly as large as the valve being replaced.

Usually, the valve structure will in a "tricuspid" form with three leaflets and three fins extending radially from three prosthetic valve commissures. In other cases, the valve may be in a "bicuspid" form with only two leaflets and two fins.

As best seen in FIGS. 12B, 13A and 13B, the leaflets 304 are generally triangular structures formed from a biological or synthetic material having a stiffness or compliance which allows the leaflets to open and close in response to hemodynamic forces when the intraluminal support structure 300 is implanted in a valve annulus or other vascular location. Suitable materials include human and animal pericardium, animal valve leaflets, Dacron^{®} (polyethylene terephthalate), PTFE (polytetrafluoroethylene), and the like. The skirt 336 and the fins 338, in contrast, are typically formed from a biological or synthetic material having a stiffness or compliance which resists deformation in response to hemodynamic forces to provide a supporting "frame" around the leaflets. The materials of the skirt 336 and the fins 338 may be the same as or different than the materials of the leaflets 334 but will be chosen or fabricated to have an increased stiffness or decreased compliance, either through different material properties, thicknesses, reinforcements, chemical treatments, or the like.

FIG. 14A is a side view of an alternative embodiment of a scaffold panel 302a of the intraluminal scaffold structure of the present invention similar to that illustrated in FIG. 14 but having a spiral spring component 328a formed in one of the longitudinal members 328 for increasing flexibility when the scaffold panel is twisted for delivery. FIG. 14A-1 is a detailed view of the spiral spring component 328a taken along line 14A-1:14A-1 in FIG. 14A. The spiral spring allows a controlled deflection of the scaffold panel 302a in a direction perpendicular to the plane of the panel to facilitate twisting of the panel.

Referring to FIG. 14B, the valve structures 304 are preferably fabricated as three separate leaflet substructures 304a-304c which may be independently attached to the coupling structures 316 and 318 and commissural posts 302 of the scaffold panel 302. Each fin 338 is divided into two opposed fin segments 338a and 338b which are brought together when attached to the scaffold panel 302, as shown in FIGS. 12B, 13A and 13B. Each leaflet substructure 304a-304c also include a skirt segment 336a, where the three segments together form the skirt 336 when the substructures are attached to the scaffold panel.

In specific embodiments, the fins 338 may be layered structures which open in response to opening of the scaffold panel 302, as shown in FIGS. 13A and 13B. The fins 338 typically assume a closed configuration, as shown in FIG. 12B, when the scaffold panel 302 is in its closed ring configuration.

To allow opening of the scaffold panel 322, one fin 338 as shown in the foreground of FIG. 13A may comprise a first layer 338a attached to the first coupling structure 316 and a second layer 338b attached to the second coupling structure 318. In this way, the entire valve structure 304 may be opened and flattened on the scaffold panel 302 when the scaffold panel is in its flattened configuration. The first layer and a second layer 338a and 338b and will close together when the free ends 308 and 310 of the scaffold panel are joined together to form the scaffold panel as a ring.

The combination of the skirt 336 and the fins 338 with the leaflets 334 provides a number of advantages. Foremost, the valve diameter provided by the leaflets may be kept relatively small, typically in a range from 27 mm to 29 mm, while the fins and skirt can extend radially outwardly to accommodate larger scaffold panel diameters needed for larger valve annuluses. The smaller valve leaflet diameter, in turn, allows the scaffold to have a lower height which is advantageous for implantation, particularly in the mitral valve. A larger valve leaflet diameter typically requires a longer or higher supporting structure, extending further into the left ventricle and increasing the risk of interfering with the aortic valve or other structures. The valve leaflet diameter reduction afforded by the present invention reduces that risk. The use of a single or limited number of valve leaflet diameters also simplifies manufacturing and inventory maintenance. The skirt and fins of the frame also have the advantage of absorbing shocks and reducing the stress on the valve leaflets. The valve is mechanically isolated and able to "float" inside the frame and is free from direct contact with the more rigid scaffold panel.

The free ends 308 and 310 of the scaffold panel 302 are joined together by the first and second coupling structures 316 and 318 which are shown in detail in FIGS. 16A to 17B. The second coupling structure 318 includes a plurality of ratchet teeth 346 which are received by a plurality of slots 348 in the first coupling structure 316. As best seen in FIGS. 17A and 17B, the ratchet teeth 346 are resiliently deflected so that they will snap into the slots 348 as the coupling structures 316 and 318 are slid over one another during deployment, as shown hereinbelow. The first and second coupling structures 316 and 318 each further include hooks 350 and catches 352 which are arranged to snap together when the coupling structures 316 and 318 are brought together in a circumferential direction. In this way, the free ends 308 and 310 are joined together to resist both circumferential and transverse separation.

As further shown in FIGS. 16A to 17B, according to the invention as claimed, each coupling structure 316 and 318 includes an attachment port 356 which is used to removably couple the scaffold panel 302 to a delivery catheter 500, as discussed in more detail below. Suture holes 358 are also provided to allow the fins 338 to be sutured to the first and second coupling structures 316 and 318 as well as to the commissural posts 320 of the scaffold panel 302.

While the first and second coupling structures 316 and 318 are presently preferred, alternative coupling structures 422 (slots) and 424 (buttons) are illustrated in FIGS. 18A and 18B and coupling structures 442 (button) and 444 (slot with gate) are illustrated in FIS. 18C and 18D. These figures also show snap-type delivery catheter couplers 426 and 446.

Referring now to FIGS. 19A and 19B, an intraluminal scaffold structure 360 comprises a panel 362 which has a flat planar construction when unstressed, as shown in FIG. 19B, and which may be stressed by folding, coiling, twisting, or the like, into a circular or ring-like configuration, as shown in FIG. 19A. Many of the features of the intraluminal scaffold structure 360 are similar to those scaffold structures described previously, including an upper crown 364, a lower crown, and a lower rail 368. The panel 362 may be closed in the circular or ring-like configuration by securing a first coupling structure 370 to a second coupling structure 372 in a manner similar to that described with respect to FIGS. 16A, 16B,17A, and 17B above. As shown in FIG. 19A the ring is only partially closed with a gap or opening 374 remaining between the free ends of the first and second coupling structures 370 and 372. A valve leaflet structure (not shown) may be attached to a pair of commissural posts 376 and the first and second coupling structures 370 and 372, as previously shown for example in FIGS. 13A and 13B. Barbs 378 are also provided.

The structure of the intraluminal scaffold structure 360 differs from those described previously in the incorporation of additional stress release features 380, typically formed as S-shaped or zig-zag elements, at multiple locations in the scaffold panel, typically at T-shaped junctions (where at three elements meet and are connected) in the structural pattern. Such additional stress relief increases the flexibility of the panel 362 which is an advantage in delivery, deployment, and performance after implantation.

Referring now to FIG. 19C, an intraluminal scaffold structure 460 comprises a panel 462 which has a flat planar construction when unstressed, as shown in FIG. 19C, and which may be stressed by folding, coiling, twisting, or the like, into a circular or ring-like configuration (not shown). Many of the features of the intraluminal scaffold structure 460 are similar to those scaffold structures described previously, including an upper crown 464, a lower crown 466, and a lower rail 468. The panel 462 may be closed in the circular or ring-like configuration by securing a first coupling structure 470 to a second coupling structure 472 in a manner similar to that described with respect to FIGS. 16A, 16B,17A, and 17B above. A valve leaflet structure (not shown) may be attached to a pair of commissural posts 476 and the first and second coupling structures 470 and 472, as previously shown for example in FIGS. 13A and 13B.

The structure of the intraluminal scaffold structure 460 is similar to that shown in FIGS. 19A and 19B as it includes stress release features 480, typically formed as S-shaped or zig-zag elements, at multiple locations in the scaffold panel, typically at T-shaped junctions (where at three elements meet and are connected) in the structural pattern. Such additional stress relief increases the flexibility of the panel 462 which is an advantage in delivery, deployment, and performance after implantation.

Referring to FIG. 19D, an intraluminal scaffold structure 560 comprises a panel 562 which has a flat planar construction when unstressed, as shown in FIG. 19C, and which may be stressed by folding, coiling, twisting, or the like, into a circular or ring-like configuration (not shown). Many of the features of the intraluminal scaffold structure 560 are similar to those scaffold structures described previously, including an upper crown 564, lower crowns 566a and 566b, and a lower rail 568. The panel 562 may be closed in the circular or ring-like configuration by securing a first coupling structure 570 to a second coupling structure 572 in a manner similar to that described with respect to FIGS. 16A, 16B, 17A, and 17B above. A valve leaflet structure (not shown) may be attached to a pair of commissural posts 576 and the first and second coupling structures 570 and 572, as previously shown for example in FIGS. 13A and 13B.

The structure of the intraluminal scaffold structure 560 is similar to that shown in FIGS. 19A, 19B and 19C as it includes stress release features 580, typically formed as S-shaped or zig-zag elements, at multiple locations in the scaffold panel, typically at T-shaped junctions (where at three elements meet and are connected) in the structural pattern. Such additional stress relief increases the flexibility of the panel 562 which is an advantage in delivery, deployment, and performance after implantation. The intraluminal scaffold structure 560 further differs from previously described embodiments in that there is no axial connection between the upper crown 564 and the the lower crowns 566a and 566b, other than the first and second coupling structure 570 and 572 and the commissural posts. Such a reduction in structure enhances flexibility but at the cost of a reduced stiffness when deployed.

Referring now to FIGS. 20 to 22, the delivery catheter 500 comprises a retractable sheath 502, an outer shaft 50, and an inner shaft 506. The retractable sheath 502, outer shaft 504, and inner shaft 506 are arranged coaxially and may be axially advanced and retracted relative to each other in order to deploy the intraluminal support structures 00 of the present invention. A distal tip 508 and a distal coupler 512 are attached to a distal end of the inner shaft 506, and a proximal coupler 510 is attached to a distal end of the outer shaft 504. The delivery catheter 500 is typically configured to be advanced over a guidewire, and the distal tip typically has a conical structure to facilitate advancement through a patient's vasculature.

An elongate carrier region 514 is formed over a distal region of the inner shaft 506 between the proximal and distal couplers 510 and 512 and is configured to receive, carry, and deploy the intraluminal support structure 300, as described in more detail below. More specifically, each of the distal and proximal couplers 510 and 512 includes an attachment block 516 which is configured to detachably secure the attachment port 356 of the first and second coupling structures 316 and 318. As best seen in FIG. 22, a retractable attachment wire 520 passes through a passage 518 and the attachment block 516 to secure the attachment port 356 to the attachment block 516. Once the intraluminal support structure 300 is placed in its desired ring configuration at a target delivery site, the attachment wire may be retracted, releasing the coupling structures 316 and 318 from the delivery catheter 500.

While the distal and proximal couplers 510 and 512 presently preferred, alternative coupling structures, such as structures 450 and 452 as shown in FIGS. 23 and 24, could also be used.

Referring now to FIGS. 25 to 29, use of the delivery catheter 500 to deliver the intraluminal structure 300 to a mitral valve MVA will be described. As shown in FIG. 25, the intraluminal support structure 300 is helically wrapped over the elongate carrier region 514 on the distal portion of the inner shaft 506. The proximal coupler 510 is releasably secured to the first coupling structure 316 of the intraluminal support structure 300 while the distal coupler 512 is releasably secured to the second coupling structure 318. The distal and proximal couplers 512 and 510 are fully separated at the distal and proximal extremities of the elongate carrier region 514, respectively, and the scaffold panel 302 is wrapped from one to three turns over the inner shaft. The full length of the intraluminal support structure 300 is covered by the retractable sheath 502.

As shown in FIGS. 26A and 26B, a distal end of the delivery catheter 500 is it advanced through the patient's left atrium over the top of the mitral valve so that it lies within the mitral valve annulus MVA. Typically, a transseptal delivery route is utilized. Once the distal end of the delivery catheter 500 has reached the valve annulus MVA, the sheath 502 will be partially retracted and the proximal coupler 510 advanced distally toward the distal coupler 512. A distal portion the helically wound scaffold panel 302 will begin to unwind and radially expand. Typically, the position of the distal coupler of 12 will be maintained (neither axially translated nor rotated) while the proximal coupler will be both axially advanced and rotated about its axis to effect and accommodate the unwinding of the helically wound scaffold panel 302.

As shown in FIGS. 27A and 27B, the retractable sheath 502 continues to be retracted while the proximal coupler 510 continues to be axially advanced and rotated to the release and unwind the scaffold panel 302. As best seen in FIG. 27B, the delivery catheter 50 positions the second coupling structure 318 perpendicularly relative to the mitral valve annulus at the location desired once the circularized scaffold is fully released with its free ends joined.

Referring now to FIGS. 28A to 28C, the proximal coupler 510 continues to be axially advanced and located until the first coupling structure 316 is brought downward over the second coupling structure 318 to engage the ratchet teeth 346 with the ratchet slots 348 and couple hooks 350 with the catches 352, as previously described with respect to FIGS. 16A to 17B. As shown in FIG. 28B, the first coupling structure 316 has been brought down over most (bit not all) of the length of the second coupling structure 318, while in FIG. 28C, the coupling structures 316 and 318 are fully engaged and locked together.

As shown in FIG. 29, the delivery catheter 500 is then removed from the mitral valve annulus MVA in the patient's heart H, and the intraluminal support structure 300 is left in place with the upper crown 322 deployed over an upper surface of the mitral valve annulus MVA and that the lower crown 324 and barbs 326 secured below the annulus. The openings 332 in the scaffold panel 302 are positioned to maintain blood flow in the left ventricle LV and to reduce interference with the adjacent aortic valve AV.

While the invention has been described with respect to a limited number of embodiment, it is to be realized that the optimum dimensional relationships for the parts of the invention, to include variations in size, materials, shape, form, function and manner of operation, assembly and use, are deemed readily apparent and obvious to one skilled in the art, and all equivalent relationships to those illustrated in the drawings and described in the specification are intended to be encompassed by the present invention.

Therefore, the foregoing is considered as illustrative only of the principles of the invention. Further, since numerous modifications and changes will readily occur to those skilled in the art, it is not described to limit the invention to the exact construction and operation shown and described and accordingly, all suitable modifications and equivalents may be resorted to, falling within the scope of the invention.

It should be noted that where reference numerals appear in the claims, such numerals are included solely or the purpose of improving the intelligibility of the claims and are no way limiting on the scope of the claims.

Having described a specific preferred embodiment of the invention with reference to the accompanying drawings, it will be appreciated that the present invention is not limited to that precise embodiment and that various changes and modifications can be effected therein by one of ordinary skill in the art without departing from the scope of the invention defined by the appended claims.

Further modifications of the invention will also occur to persons skilled in the art and all such are deemed to fall within the scope of the invention as defined by the appended claims.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

Citation or identification of any reference in this application shall not be construed as an admission that such reference is available as prior art to the invention.

Section headings are used herein to ease understanding of the specification and should not be construed as necessarily limiting.

While the invention has been described with respect to a limited number of embodiments, it will be appreciated that many variations, modifications and other applications of the invention may be made.

## Claims

1. An intraluminal support structure (300) comprising:
a scaffold panel (302, 400) having a first free and a second free end (308, 310) with a longitudinal axis (312) therebetween with when flattened;
wherein the scaffold panel is configured to be helically wrapped over an elongated carrier region (514) of a delivery catheter (500);
wherein the first and second free ends are configured to be coupled to each other to form the scaffold panel into a ring when the scaffold panel is deployed from the elongated carrier region of the delivery catheter; and
wherein the first free end of the scaffold panel comprises a first attachment structure and the second free end of the scaffold panel comprises a second attachment structure, wherein the first and second attachment structures are configured to releasably attach the scaffold panel to the elongated carrier region of the delivery catheter when the scaffold panel is helically wrapped over the elongated carrier region of the delivery catheter.

2. The intraluminal support structure (300) of claim 1, wherein the scaffold panel is pre-shaped as a ring or as a flat panel.

3. The intraluminal support structure (300) of any one of claims 1 to 2, wherein the first free end of the scaffold panel comprises a first coupling structure and the second free end of the scaffold panel comprises a second coupling structure, wherein the first and second coupling structures are configured to mate with each other to form the ring when the scaffold panel is deployed from the elongated carrier region of the delivery catheter.

4. The intraluminal support structure (300) of claim 3, wherein the first and second coupling structures are configured to be coupled by axial engagement controlled by the delivery catheter, optionally wherein the first and second coupling structures comprise one or more of slots, hooks, buttons, snaps, and ratchets.

5. The intraluminal support structure (300) of any one of claims 3 to 4, further comprising a leaflet structure attached to an inner surface of the scaffold panel, optionally wherein the leaflet structure comprises a plurality of leaflets connected at plurality of commissures.

6. The intraluminal support structure (300) of claim 5, wherein at least one of the leaflet commissures is split with a first segment attached to the first coupling structure and a second segment attached to the second coupling structure, optionally wherein the first segment and the second segment are separated when the scaffold panel is in an open and are configured to be brought together when the first and second coupling structures are attached to form the scaffold panel into the ring.

7. The intraluminal support structure (300) of claim 6, wherein the remaining leaflet commissures are fixed to posts disposed laterally across the inner face of the scaffold panel.

8. The intraluminal support structure (300) of claims 6 or 7, wherein the leaflet structure comprises a plurality of coapting leaflets centrally positioned in a frame, wherein the leaflets are sufficiently pliable to open and close in response to hemodynamic forces and wherein the frame is sufficiently stiff to resist deformation resulting from hemodynamic forces after implantation.

9. The intraluminal support structure (300) of claim 8, wherein the frame comprises a skirt having an outer periphery attached to an upstream end of the scaffold panel and plurality of fins extending radially outwardly from the commissures of the leaflets, wherein one of the fins is split into segments which attach the first and second segments of the split commissure to the first and second coupling structures of the scaffold panel.

10. The intraluminal support structure (300) of any one of claims 5 to 9, wherein the scaffold panel has a width in a lateral direction in a range from 6 mm to 30 mm and the ring has a diameter in a range from 30 mm to 60 mm when formed.

11. The intraluminal support structure (300) of claim 10, wherein leaflets have a diameter in a range from 27 mm to 29 mm when the ring is formed.

12. The intraluminal support structure (300) of claim 1, wherein the first and second attachment structures comprise one or more of slots, hooks, buttons, snaps, ratchets.

13. A system for implanting a support structure in a valve annulus, said system comprising:
an intraluminal support structure (300) as in any one of claims 1 to 12;
a delivery catheter (500) having a proximal end, a distal end, and an elongated carrier region near the distal end; and
a distal coupler (512) configured to releasably attach the free first end of the scaffold panel and a proximal coupler configured to releasably attach the second end of the scaffold panel, wherein the distal and proximal couplers are configured to releasably hold the scaffold panel in a helically wrapped configuration within the elongated carrier region.

14. The system as in claim 13, wherein the distal coupler is configured to both translate along and rotate about a longitudinal axis of the catheter, optionally wherein the delivery catheter comprises an inner housing and an inner shaft coaxially disposed within a central passage of the outer shaft, wherein the inner housing is mounted to both translate and rotate within the central passage.

15. The system as in claim 14, wherein the distal coupler is carried on a distal region of the inner shaft and the proximal coupler is carried on a distal region of the inner housing.

## Patentansprüche

1. Eine intraluminale Stützstruktur (300), die Folgendes umfasst:
eine Gerüstplatte (302, 400), die ein erstes freies und ein zweites freies Ende (308, 310) aufweist, mit einer dazwischenliegenden Längsachse (312), wenn sie flach liegt;
wobei die Gerüstplatte dazu ausgelegt ist, spiralförmig über eine längliche Trägerregion (514) eines Zuführkatheters (500) gewickelt zu werden;
wobei das erste und zweite freie Ende dazu ausgelegt sind, miteinander gekoppelt zu werden, um die Gerüstplatte zu einem Ring zu formen, wenn die Gerüstplatte von der länglichen Trägerregion des Zuführkatheters abgelegt wird; und
wobei das erste freie Ende der Gerüstplatte eine erste Befestigungsstruktur umfasst und das zweite freie Ende der Gerüstplatte eine zweite Befestigungsstruktur umfasst, wobei die erste und zweite Befestigungsstruktur dazu ausgelegt sind, die Gerüstplatte lösbar an der länglichen Trägerregion des Zuführkatheters zu befestigen, wenn die Gerüstplatte spiralförmig über die längliche Trägerregion des Zuführkatheters gewickelt wird.

2. Intraluminale Stützstruktur (300) nach Anspruch 1, wobei die Gerüstplatte als ein Ring oder als eine flache Platte vorgeformt ist.

3. Intraluminale Stützstruktur (300) nach einem der Ansprüche 1 bis 2, wobei das erste freie Ende der Gerüstplatte eine erste Kopplungsstruktur umfasst und das zweite freie Ende der Gerüstplatte eine zweite Kopplungsstruktur umfasst, wobei die erste und zweite Kopplungsstruktur dazu ausgelegt sind, zueinander zu passen, um den Ring zu bilden, wenn die Gerüstplatte von der länglichen Trägerregion des Zuführkatheters abgelegt wird.

4. Intraluminale Stützstruktur (300) nach Anspruch 3, wobei die erste und zweite Kopplungsstruktur dazu ausgelegt sind, durch eine von dem Zuführkatheter gesteuerten axiale Eingriffnahme gekoppelt zu werden, wobei die erste und zweite Kopplungsstruktur optional eines oder mehrere von Schlitzen, Haken, Knöpfen, Schnappverschlüssen und Sperrklinken umfassen.

5. Intraluminale Stützstruktur (300) nach einem der Ansprüche 3 bis 4, die ferner eine Segelstruktur umfasst, die an einer inneren Oberfläche der Gerüstplatte befestigt ist, wobei die Segelstruktur optional eine Vielzahl von Segeln umfasst, die mit einer Vielzahl von Kommissuren verbunden sind.

6. Intraluminale Stützstruktur (300) nach Anspruch 5, wobei mindestens eine der Segelkommissuren geteilt ist, mit einem ersten Segment, das an der ersten Kopplungsstruktur befestigt ist, und einem zweiten Segment, das an der zweiten Kopplungsstruktur befestigt ist, wobei das erste Segment und das zweite Segment optional getrennt sind, wenn die Gerüstplatte in einem offen ist, und dazu ausgelegt sind, zusammengebracht zu werden, wenn die erste und zweite Kopplungsstruktur befestigt werden, um die Gerüstplatte zu dem Ring zu formen.

7. Intraluminale Stützstruktur (300) nach Anspruch 6, wobei die verbleibenden Segelkommissuren an Pfosten angebracht sind, die lateral über die innere Fläche der Gerüstplatte angeordnet sind.

8. Intraluminale Stützstruktur (300) nach Ansprüchen 6 oder 7, wobei die Segelstruktur eine Vielzahl von aneinanderpassenden Segeln umfasst, die zentral in einem Rahmen positioniert sind, wobei die Segel ausreichend nachgiebig sind, um sich als Reaktion auf hämodynamische Kräfte zu öffnen und zu schließen, und wobei der Rahmen ausreichend starr ist, um einer nach der Implantation aus hämodynamischen Kräften resultierenden Verformung zu widerstehen.

9. Intraluminale Stützstruktur (300) nach Anspruch 8, wobei der Rahmen eine Schürze, die eine äußere Peripherie aufweist, die an einem stromaufwärts gelegenen Ende der Gerüstplatte befestigt ist, und eine Vielzahl von Rippen, die sich radial von den Kommissuren der Segel nach außen erstrecken, umfasst, wobei eine der Rippen in Segmente aufgeteilt ist, die das erste und zweite Segment der geteilten Kommissur an der ersten und zweiten Kopplungsstruktur der Gerüstplatte befestigen.

10. Intraluminale Stützstruktur (300) nach einem der Ansprüche 5 bis 9, wobei die Gerüstplatte eine Breite in einer lateralen Richtung in einem Bereich von 6 mm bis 30 mm aufweist und der Ring einen Durchmesser in einem Bereich von 30 mm bis 60 mm aufweist, wenn er geformt worden ist.

11. Intraluminale Stützstruktur (300) nach Anspruch 10, wobei die Segel einen Durchmesser in einem Bereich von 27 mm bis 29 mm aufweisen, wenn der Ring geformt worden ist.

12. Intraluminale Stützstruktur (300) nach Anspruch 1, wobei die erste und zweite Befestigungsstruktur eines oder mehrere von Schlitzen, Haken, Knöpfen, Schnappverschlüssen und Sperrklinken umfassen.

13. Ein System zur Implantation einer Stützstruktur in einem Klappenannulus, wobei das genannte System Folgendes umfasst:
eine intraluminale Stützstruktur (300) nach einem der Ansprüche 1 bis 12;
einen Zuführkatheter (500) mit einem proximalen Ende, einem distalen Ende und einer länglichen Trägerregion in der Nähe des distalen Endes; und
einen distalen Koppler (512), der zum lösbaren Befestigen des freien ersten Endes der Gerüstplatte ausgelegt ist, und einen proximalen Koppler, der zum lösbaren Befestigen des zweiten Endes der Gerüstplatte ausgelegt ist, wobei der distale und proximale Koppler dazu ausgelegt sind, die Gerüstplatte lösbar in einer spiralförmig gewickelten Konfiguration innerhalb der länglichen Trägerregion zu halten.

14. System nach Anspruch 13, wobei der distale Koppler dazu ausgelegt ist, sowohl entlang einer Längsachse des Katheters verschoben zu werden als auch um sie zu rotieren, wobei der Zuführkatheter optional ein inneres Gehäuse und einen inneren Schaft umfasst, der koaxial innerhalb eines zentralen Durchgangs des äußeren Schafts angeordnet ist, wobei das innere Gehäuse sowohl zum Verschieben als auch Rotieren innerhalb des zentralen Durchgangs montiert ist.

15. System nach Anspruch 14, wobei der distale Koppler auf einer distalen Region des inneren Schafts getragen wird und der proximale Koppler auf einer distalen Region des inneren Gehäuses getragen wird.

## Revendications

1. Structure de support intraluminale (300) comprenant :
un panneau de support (302, 400) ayant une première extrémité libre et une deuxième extrémité libre (308, 310) avec un axe longitudinal (312) entre celles-ci dans la configuration aplatie ;
dans laquelle le panneau de support est configuré pour être enroulé de manière hélicoïdale par-dessus une région de support allongée (514) d'un cathéter de mise en place (500) ;
dans laquelle les première et deuxième extrémités libres sont configurées pour être couplées l'une à l'autre afin de former un anneau à partir du panneau de support lorsque le panneau de support est déployé à partir de la région de support allongée du cathéter de mise en place ; et
dans laquelle la première extrémité libre du panneau de support comprend une première structure de fixation et la deuxième extrémité libre du panneau de support comprend une deuxième structure de fixation, les première et deuxième structures de fixation étant configurées pour fixer de manière amovible le panneau de support à la région de support allongée du cathéter de mise en place lorsque le panneau de support est enroulé de manière hélicoïdale par-dessus la région de support allongée du cathéter de mise en place.

2. Structure de support intraluminale (300) selon la revendication 1, dans laquelle le panneau de support est préformé sous la forme d'un anneau ou sous la forme d'un panneau plat.

3. Structure de support intraluminale (300) selon l'une quelconque des revendications 1 ou 2, dans laquelle la première extrémité libre du panneau de support comprend une première structure de couplage et la deuxième extrémité libre du panneau de support comprend une deuxième structure de couplage, les première et deuxième structures de couplage étant configurées pour se coupler l'une à l'autre afin de former l'anneau lorsque le panneau de support est déployé à partir de la région de support allongée du cathéter de mise en place.

4. Structure de support intraluminale (300) selon la revendication 3, dans laquelle les première et deuxième structures de couplage sont configurées pour être couplées par une mise en prise axiale contrôlée par le cathéter de mise en place, optionnellement dans laquelle les première et deuxième structures de couplage comprennent un ou plusieurs de fentes, de crochets, de boutons, de boutons à pression, et de cliquets.

5. Structure de support intraluminale (300) selon l'une quelconque des revendications 3 à 4, comprenant en outre une structure en feuillets fixée à une surface interne du panneau de support, optionnellement dans laquelle la structure en feuillets comprend une pluralité de feuillets reliés à une pluralité de commissures.

6. Structure de support intraluminale (300) selon la revendication 5, dans laquelle au moins l'une des commissures de feuillets est divisée, un premier segment étant fixé à la première structure de couplage et un deuxième segment étant fixé à la deuxième structure de couplage, optionnellement dans laquelle le premier segment et le deuxième segment sont séparés lorsque le panneau de support est dans un ouvert et sont configurés pour être réunis lorsque les première et deuxième structures de couplage sont fixées pour former l'anneau à partir du panneau de support.

7. Structure de support intraluminale (300) selon la revendication 6, dans laquelle les commissures de feuillets restantes sont fixées à des montants disposés latéralement en travers de la face interne du panneau de support.

8. Structure de support intraluminale (300) selon les revendications 6 ou 7, dans laquelle la structure en feuillets comprend une pluralité de feuillets en coaptation positionnés centralement dans un cadre, les feuillets étant suffisamment souples pour s'ouvrir et se refermer en réponse à des forces hémodynamiques et le cadre étant suffisamment rigide pour résister à une déformation résultant des forces hémodynamiques après l'implantation.

9. Structure de support intraluminale (300) selon la revendication 8, dans laquelle le cadre comprend une jupe dont une périphérie externe est fixée à une extrémité amont du panneau de support et une pluralité d'ailettes s'étendant radialement vers l'extérieur à partir des commissures des feuillets, l'une des ailettes étant divisée en segments qui fixent les premier et deuxième segments de la commissure fendue aux première et deuxième structures de couplage du panneau de support.

10. Structure de support intraluminale (300) selon l'une quelconque des revendications 5 à 9, dans laquelle le panneau de support a une largeur dans une direction latérale dans une plage de 6 mm à 30 mm et l'anneau a un diamètre dans une plage de 30 mm à 60 mm lorsqu'il est formé.

11. Structure de support intraluminale (300) selon la revendication 10, dans laquelle les feuillets ont un diamètre dans une plage de 27 mm à 29 mm lorsque l'anneau est formé.

12. Structure de support intraluminale (300) selon la revendication 1, dans laquelle les première et deuxième structures de fixation comprennent un ou plusieurs de fentes, de crochets, de boutons, de boutons à pression, et de cliquets.

13. Système destiné à l'implantation d'une structure de support dans un anneau valvulaire, ledit système comprenant :
une structure de support intraluminale (300) selon l'une quelconque des revendications 1 à 12 ;
un cathéter de mise en place (500) ayant une extrémité proximale, une extrémité distale, et une région de support allongée près de l'extrémité distale ; et
un élément de couplage distal (512) configuré pour fixer de manière amovible la première extrémité libre du panneau de support et un élément de couplage proximal configuré pour fixer de manière amovible la deuxième extrémité du panneau de support, les éléments de couplage distal et proximal étant configurés pour maintenir de manière amovible le panneau de support dans une configuration enroulée de manière hélicoïdale à l'intérieur de la région de support allongée.

14. Système selon la revendication 13, dans lequel l'élément de couplage distal est configuré pour à la fois se déplacer en translation le long d'un axe longitudinal du cathéter et pivoter autour dudit axe, optionnellement dans lequel le cathéter de mise en place comprend un logement interne et une tige interne disposés coaxialement à l'intérieur d'un passage central de la tige externe, le logement interne étant monté pour à la fois se déplacer en translation et pivoter à l'intérieur du passage central.

15. Système selon la revendication 14, dans lequel l'élément de couplage distal est porté sur une région distale de la tige interne et l'élément de couplage proximal est porté sur une région distale du logement interne.
